# EUROPEAN PATENT SPECIFICATION

(11) **EP 1 897 882 B1**
(45) Date of publication and mention of the grant of the patent: **10.12.2014**
(21) Application number: 06705534.3
(22) Date of filing: 23.01.2006
(51) Int. Cl.: C07D 473/16, C07H 19/16, C07H 19/20, A61K 31/52, A61K 31/7076, A61P 35/00

(54) **N2-QUINOLYL OR ISOQUINOLYL SUBSTITUTED PURINE DERIVATIVES, THE PREPARATION AND USES THEREOF**
N2-CHINOLYL- ODER -ISOCHINOLYLSUBSTITUIERTE PURINDERIVATE, DEREN HERSTELLUNG UND DEREN VERWENDUNG
DÉRIVÉS DE PURINE SUBSTITUÉE PAR UN GROUPE N2-QUINOLÉYLE OU ISOQUINOLÉYLE, LEURS PRÉPARATIONS ET UTILISATIONS

(30) Priority: 16.06.2005 CN 200510026846
(43) Date of publication of application: 12.03.2008
(73) Proprietor: Zhe Jiang Medecine Co., Ltd. Xinchang Pharmaceutical Factory, Zhejiang 312500 (CN)
(72) Inventor: WU, Zhanggui, Hangzhou, Zhejiang 310006 (CN)
(74) Representative: Meyer-Dulheuer, Karl-Hermann
(86) International application number: PCT/CN2006/000113
(87) International publication number: WO 2006/133611

(56) References cited:
- EP-A- 0 704 215
- WO-A-01/09134
- WO-A-2005/016528
- WO-A-2005/097135
- WO-A1-00/55161
- JP-A- 2003 055 377
- JP-A- 2003 119 197
- US-A- 4 853 386

## Description

The present invention relates to pharmaceutical chemistry, and particularly to N²-quinoline- or isoquinoline-substituted purine derivatives, processes for their production, pharmaceutical compositions comprising the same, and methods for using these derivatives in the treatment of cancer.

Cancer remains a major threat to human health. Most human cancers are caused by environment factors, and not fewer than 5 million people die of cancer per year worldwide. Although many methods of treatment, such as surgery, radiation therapy, chemotherapy, etc., are available, curative ratio is generally low. Presently, the use of pharmaceuticals remains one of the most effective cancer prevention and therapy method.

Purine or pyridine derivatives are known to have anti-viral or anti-cancer activities. See, e.g., EP 173624, EP 253412, EP 353955, WO 9201698, and EP 481214, etc.

In natural or synthetic nucleotide derivatives, the pyridine, purine or other heterocylic substituent is located on the 1 position of the saccharide ring (corresponding to the 2 position of a hydroxyl-substituted tetrahydrofuran derivative). These compounds have recently been reported to have anti-cancer or antiviral activities.

Another group of derivatives, O⁶-alkyl purine derivatives, are known for their ability to inhibit the activity of O⁶-alkylguanine-DNA alkyltransferase (AGT), thereby enhancing the effectiveness of alkylating agents as tumor chemotherapeuticals.

Although the cancer cell killing mechanism of the O⁶-methyl guanine in the ATase defect cell is not clear, it is generally understood that the mechanism of the O⁶- chloroethyl guanine includes a cycloethidene guanine intermediate, which cross links the DNA double strand in the cytidine residue. This linkage can be eliminated or prevented via an ATase mediated dechloroethylation or acomplex formal. Methods for antagonizing O⁶-alkylguanine-DNA alkyl transferase in tumor cells have been reported, e.g., in U.S. Patent No. 5,091,430 and WO 9113898.

N²-substituted purine derivatives are known. For example, N⁶-disubstituted purine derivatives which can be used to treat allergies are described in U.S. Patent No. 4,853,386. 6-cyclopropylamino-9H purine derivatives are described in JP 2003-55377A and JP 2003-119197A, which have antiviral properties. Glycosylated purine derivatives which have antiinflammatory properties are described in J. Org. Chem (2004, 69:3212-3215). N²-butylphenyl-2'-desoxypurine derivatives are described in J. Med. Chem (1984, 27:175-181), which have the activity of inhibiting eukaryotic DNA α polymerase. 2, 6, 9-substituted purine derivatives are described in Tetrahedron Letters (1998, 39:1827-1830).The above mentioned purine compounds have partially the same or similar structure with the compounds of this invention, but none of the above mentioned compounds, however, have been shown to have anti-cancer activities or the ability to inhibit abnormal cell growth. Accordingly, there is a need for discovery of anti-tumor drugs that may be used as anti-cancer agents, with low toxicity, wide anti-cancer spectrum, high anti-cancer activity, and high stability.

WO 2005/016528 A2 discloses a class of purine derivatives and uses of these derivatives to treat or prevent diseases or disorders associated with cSRC, Lck, FGFR3, Flt3, TrkB, Bmx, and/or PFGFRα kinase activity.

The present invention relates to highly stable N²-substituted purine derivatives with low toxicity wide anti-cancer spectrum and high anti-cancer activities.

The present invention relates to compounds having the following formula A of N²-quinoline- or isoquinoline-substituted purine compounds, their salts, or hydrates.

The invention features a compound having the following formula A, its salts, or hydrates: 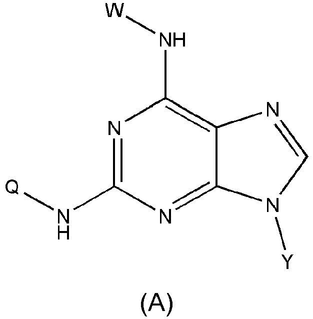 wherein
Y represents a hydrogen, or a pharmaceutically-acceptable saccharide of any one of the following formulas: 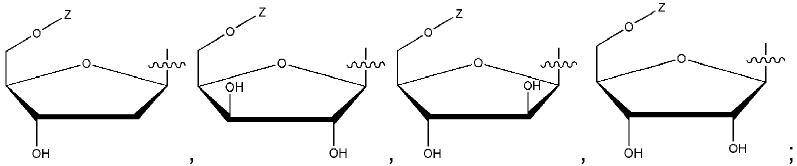
Z represents a hydrogen or a substituent having any one of the following formulas: 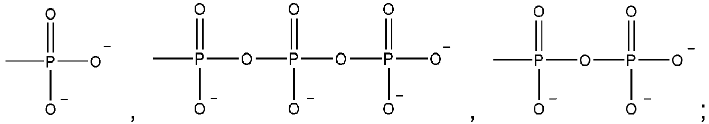
Q represents a substituent having any one of the following formulas: 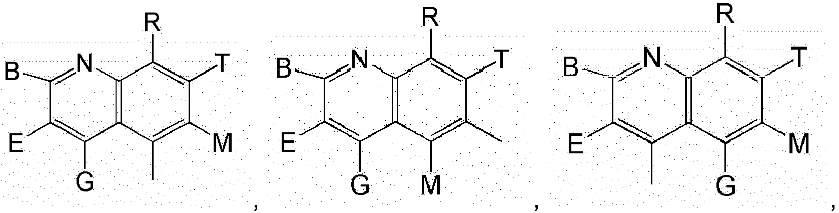 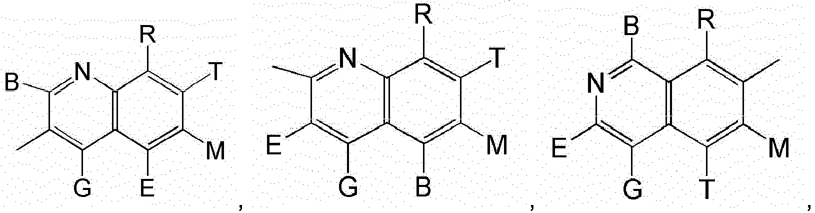 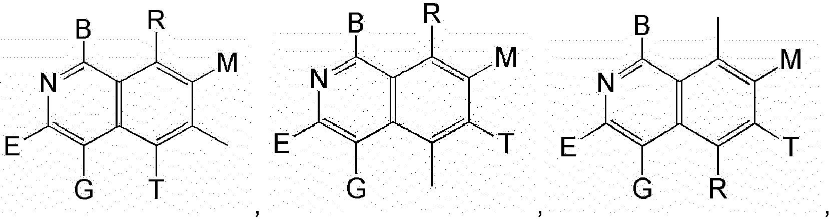 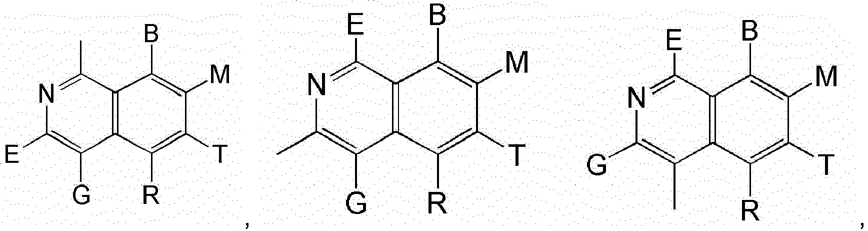
B, E, G, R, T, and M each independently represent hydrogen, a straight or branched C₁₋₆ alkyl or haloalkyl, a C₃₋₆ cycloalkyl, a halogen, a cyano, or an amino; and
W represents a substituent having any one of the following formulas:

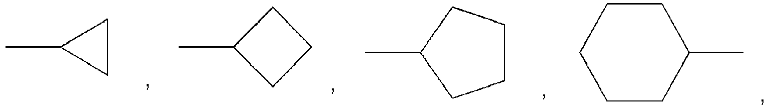

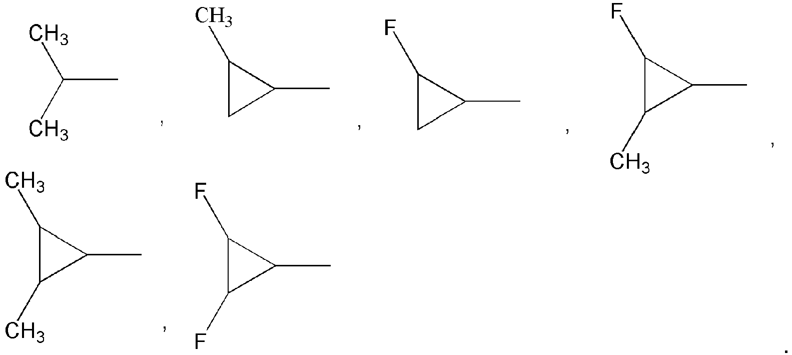

More preferably, W is one of:

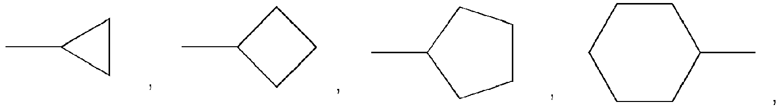

Still more preferably, W represents:

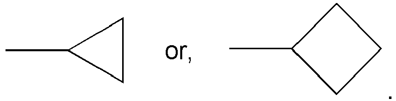

In one preferred embodiment, in the compound of the present invention having Formula A, Q represents:

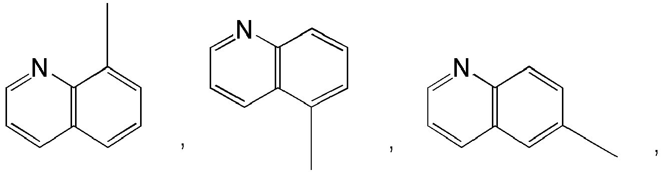

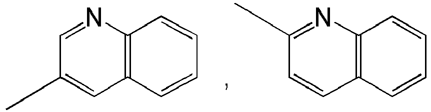

More preferably, Q represents the following group:

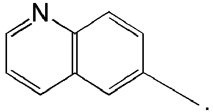

In one preferred embodiment, in the compound of the present invention having Formula A, the substituent B, E, G, R, T, or M each independently represents a hydrogen, a fluorine, a methyl, a trifluoromethyl, a cyano, or an amino, especially a hydrogen.

In one preferred embodiment, in the compound of the present invention having Formula A, Y is a hydrogen.

The following specific compounds are particularly preferred:

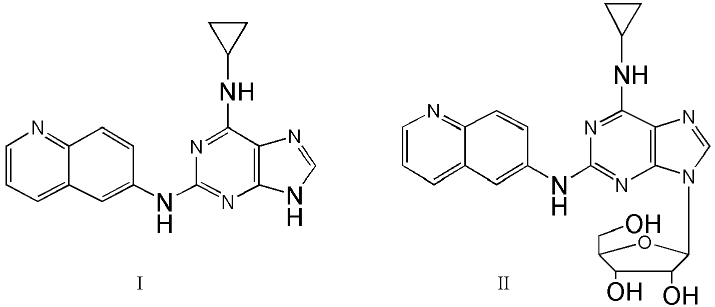

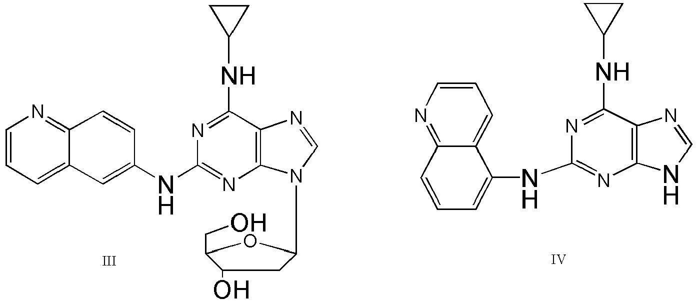

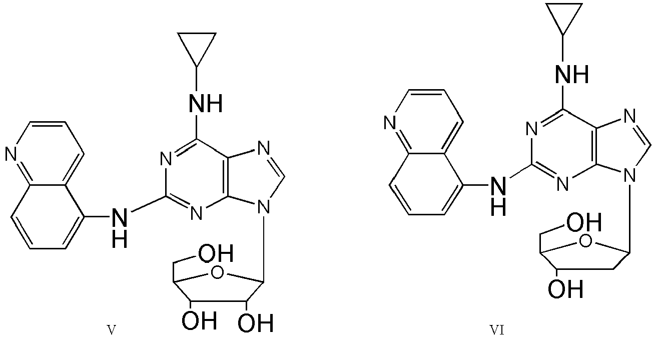

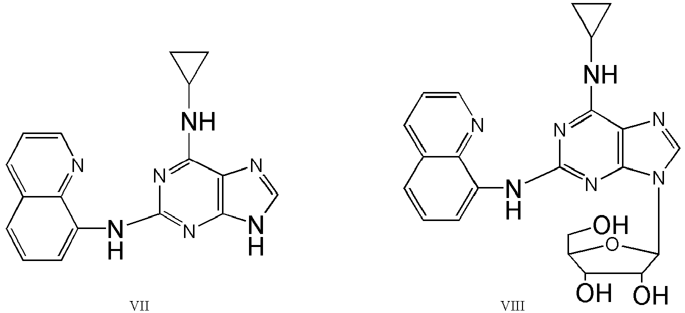

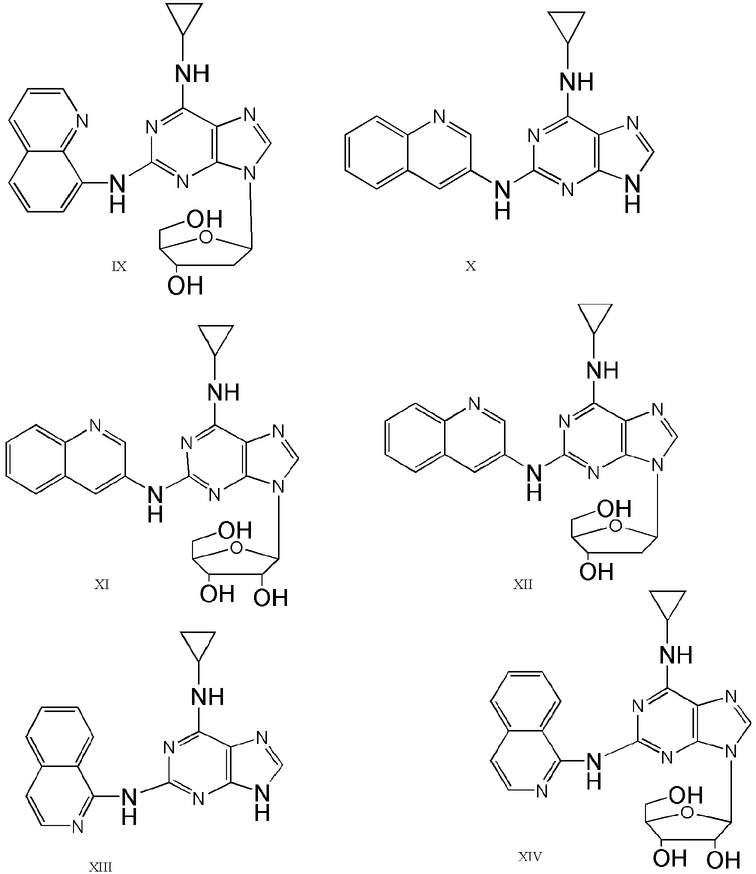

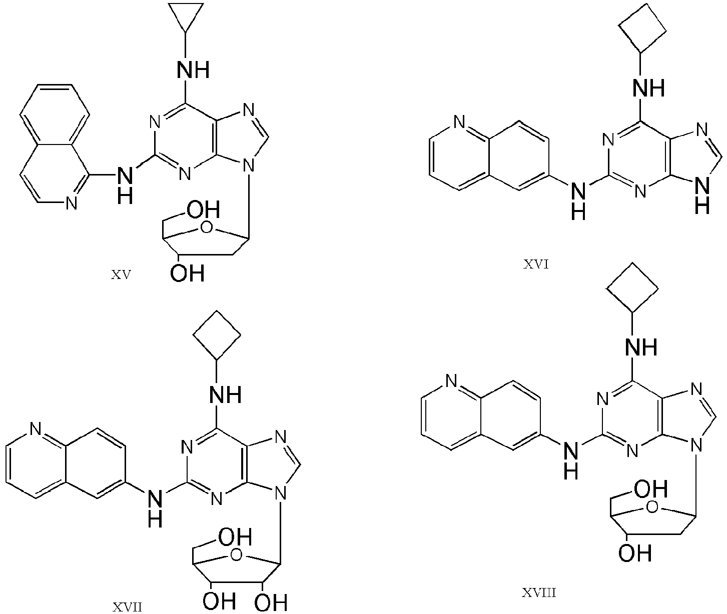

Compound (I) is especially preferred:

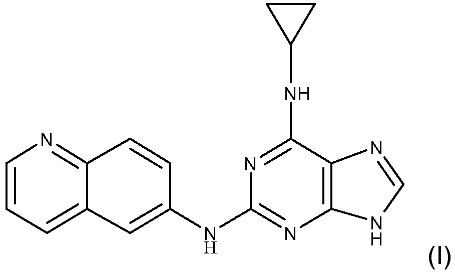

The another propose of this invention is to provide with a pharmaceutical composition of compounds of formula I to XVIII, the composition being composed by one of any compound or its salts or hydrates in formula I to XVIII with pharmaceutical adjuvants. The described composition is suitable for intestinal, local or parenteral administration, or a pharmaceutical composition for the administration of mammalian animals via inhalation. The compositions can be tablets, capsules, pills, oral liquid preparations, granules, powders, injections, implants or preparations for external use.

The present invention further provides with a process for the production of the above described compounds or its salts or hydrates. In one embodiment, the compounds of this invention can be prepared in accordance with the following schemes:
1). The reaction is carried out in the presence of formula (j) compound and Q-NH₂, 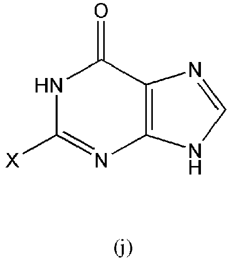 resulting in formula (b) compound 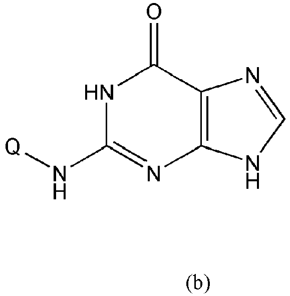 The above reaction is carried out in an organic solvent, in the presence of formula (j) compound and 0.8-1.5 mol/ml Q-NH₂, the mixture was heated at 50-150°C to react for 1-72 hours, then water was added to the reaction mixture, and the reaction mixture was allowed to cool to room temperature.
2). Preparation of formula (c) compound with formula (b) compound 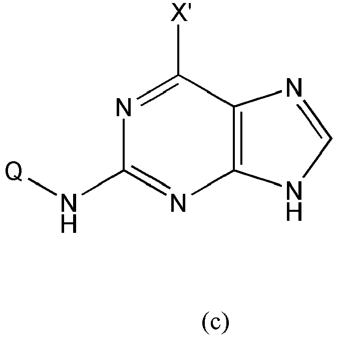 The reaction is carried out in an organic solvent, in the presence of formula (b) compound and a halogenating agent, the mixture was heated at 50-150 °C to react for 1-72 hours, cooled. Water was added and the pH of the mixture ph was adjusted to 2-5 with an acid, and was allowed to cool at room temperature.
3). Preparation of formula (f) compound by react formula (c) compound with W-N H₂ 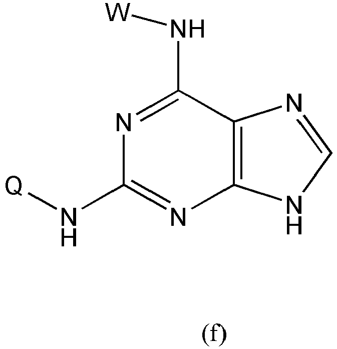 The reaction is carried out in an organic solvent, in the presence of formula (c) compound, 0.8-1.5 mol/mol W-NH₂ and an acid acceptor, the mixture was heated at 50-150°C to react for 1-72 hours. The solvent was then distilled off.
   X represents Br, X' represents Cl, W is as defined above.

According to another embodiment, the present invention also provides a process for the production of the above mentioned compound or its salts, the compounds of this invention can be prepared in accordance with the following schemes:
1) The reaction is carried out in the presence of to formula (k) compound and W-NH₂, 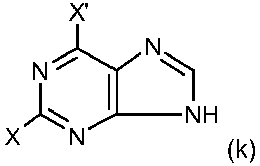 resulting in formula (e) compound: 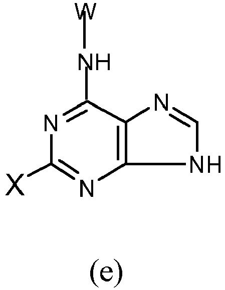 The reaction is carried out in an organic solvent, in the presence of formula (k) compound , 0.8-1.5mol/mol W-NH₂ and an acid acceptor, the mixture was heated at 30-120°C to react for 1-72 hours. The solvent was then distilled off.
2) Preparation of formula (f) compound by react formula (e) compound with Q-NH₂ 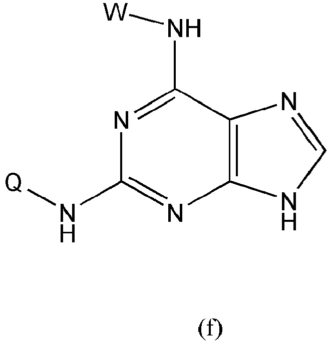 The reaction is carried out in an organic solvent, in the presence of formula (e) compound, 0.8-1.5mol/mol Q-N H₂, and an acid acceptor. The mixture was heated at 70-170 °C to react for 1-72 hours. The solvent was then distilled off.
   X and X' represent Cl, and W is as defined above.

The salt of the compound of Formula A can be any pharmaceutically acceptable salt, known to those skilled in the art. The addition salt of the compound can be synthesized by inorganic acid or organic acid, preferably as hydrochloride, hydrobromide, hydroiodide, p-toluenesulfonate, phosphate, sulfate, perchloride, acetate, trifluoroacetate, propionate, citrate, malonate, succinate, lactate, oxalate, tartrate, benzoate. The salt also can be formed by a base, including an inorganic or organic base, alkali earth metal salts such as magnesium salts, calcium salts, organic amines such as morpholine, piperidine, dimethylamine, diethylamine, etc.

In a preferred embodiment, the compounds of this invention are prepared in accordance with the following routines A and B:
Route A: 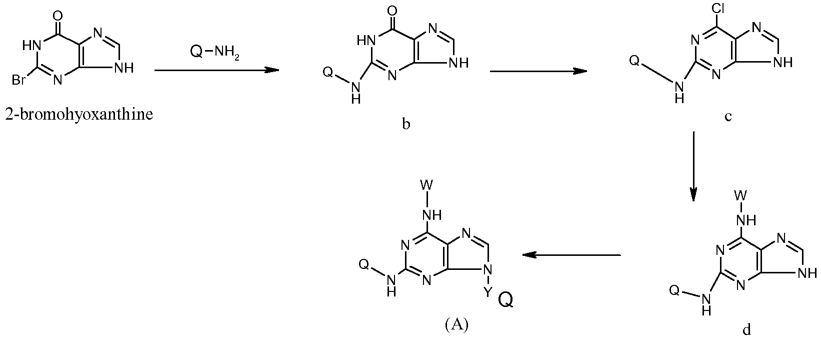
Route B:

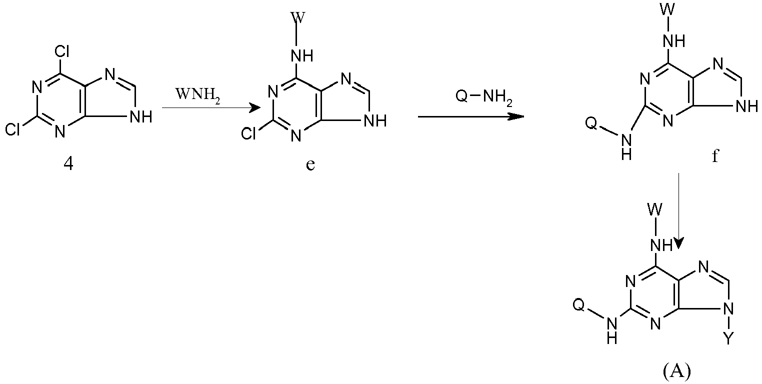

The pharmaceutical composition of the present invention may be administered to mammals (include humans) internally (such as orally or via rectal administration), local, or parenterally or by inhalation spray, such as oral, injection, implantation, or external form. Suitable formulations for oral include tablets (such as conventional tablets, buccal tablets, sublingual tablet, oral cavity patch, chewable tablet, dispersible tablet, soluble tablet, effervescent tablet, vaginal tablet, vaginal effervescent tablet, sustained-release tablet, controlled release tablet, enteric coated tablet, buccal rapid-release tablet), capsules (hard capsules, soft capsules, sustained-release capsules, controlled-release capsules, enteric-coated capsules, etc), pills (dripping pills, sugar coated pills, pellets), oral liquid preparation (syrups oral solution, oral suspension, oral emulsion, etc), granules (suspension granules, effervescent granules, enteric coated granules, sustained-release granules, controlled-release granules, etc), and powders. Injections including injectable solution, sterile, powder for injection, or sterile solids(including preparation produced by solvent crystallization, spray dry or lyophilization technologies etc.) concentrated solution for injection; implants; etc, and other external medicament forms such as suppositories, aerosol, aerosol powder, spray, pellicles, gel, patches, etc.

Pharmaceutical composition of the present invention may be prepared according to any public known dosage form preparation method in the field of pharmaceutical composition. It is suitable for treating cancers and the related diseases, and may be used alone or in combination with other one or more anti-cancer drugs. The quantity of active ingredient composited with carrier substance to prepare single dosage form can be varied according to the treated hosts and types of administration.

The compounds of this invention can be used to prevent or treat abnormal cell proliferation, especially those found in tumors or cancers, including lung cancer, liver cancer, and leukemia,.

It was discovered from the determination of compounds in this invention in the experiments for inhibition the growth of tumor cell in vitro, that this compound shows remarkable inhibition action to the growth of human cells of lung cancer, liver cancer and leukemia incubated in vitro, and also showed dose-response effect relationship. The better formula I compound shows IC₅₀=1 1.22µg/ml to human liver cancer, IC₅₀=12.8µg/ml to human leukemia cells (blood cancer) and IC₅₀=10.24µg/ml of human lung cancer cells. When administered the pharmaceutical composition of the invention by intraperitonial injection to mouse, the LD₅₀ of mouse is 160mg/kg.

In the tumor inhibition experiment to liver cancer of H₂₂ mouse 80mg/kg, the inhibition rate of this drugs is 69%.

The compound can be applied to treat tumor in association with chemotherapy, radiotherapy and biochemical therapy to enhance effect and lowering pharmaceutical side effects.

### Pharmacological Experiments

The activity of compounds has been determined with compound I as example:

### Compound I:

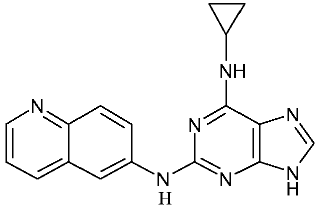

### 1. Determination of IC₅₀ (Lewis lung cancer).

(a) Incubated the Lewis lung cancer cells in DMEM nutrition solution containing 15% bovine serum, inoculate into 96-holer inoculation plate with 1×10⁴ cells/hole, placed the plate in a 37°C 5% CO₂ incubator. Diluted the compound to suitable concentration with DMEM incubation solution which containing 15% bovine serum. Added the diluted solution into each hole of 96 holes plate, 3 holes for each concentration and 6 holes for blank control, lucubrated for 72 hours, MTT solution was added and allowed to stand for 1 hour, added DMSO to develop the color, the OD value was determined with Enzyme Label instrument. Calculated the killing rate of each concentration, and calculated IC₅₀ value according to coordinate method.
   Lewis lung cancer: IC₅₀=10.24µg/ml.
(b) Determined the IC₅₀=11.22µg/ml of compound I for human liver cancer cells with the same of above mentioned testing method.
(c) Determined the IC₅₀=12.8µg/ml of compound I for human leukemia cells (blood cancer) with the same of above mentioned testing method. It showed that this compound has the ability to inhibit the growth of non solid tumor cells.

### 2. Remarkable antitumor activity

(a) Divided randomly 30 mice (Kunming special) of a equal numbers of male and female of 21-22g body weight into 3 groups, each group consisted 10 mice. Inoculated liver cancer H₂₂ cells suspension 0.2ml for each mouse subcutaneous by at the right side of abdomen. The next day, intraperitoneally injected separately 80mg and 40mg/kg of this compound into 2 groups of mice once a day, continuously for 7days. Injected the control group IP with DMSO + normal saline. Killed the mice at the following day after stopping the administration, weighed the body weigh and tumor weigh, and calculated the tumor inhibition rate. The compound possessed obvious antitumor activity to H₂₂, with tumor inhibition rate of 69% and 40% with dosage of 80 mg and 40 mg/kg respectively.

**Table 1. Inhibition rate for liver cancer of compound I. Class body weight(g) tumor weight(g) inhibition rate(%)**

| Before | | after | | |
|---|---|---|---|---|
| 80mg/kg | 20.6 | 21.4 | 0.52 | 69 |
| 40mg/kg | 21.9 | 25.8 | 0.99 | 40 |
| Control | 21.3 | 29.7 | 1.62 | |

(b) Inhibition of cancer cells in vitro
Treated human lung cancer cells (Lewis) and leukemia cells with 8µg/m, 16µg/ml of compound I, observed the dynamic growth of cells for 4 days. The difference between medicated and control groups is small within two days, at beginning of third day, the cells number of medicated group dropped rapidly, and the cancer cells in control group were continuously growing logarithmically The difference between groups were increasing with time prolongation.

The above pharmacological experiments show that the compound mentioned in this invention possesses obvious growth inhibition effect to the liver cancer cells, leukemic cells (solid tumor and non-solid tumor), and with dose-response relationship.

### Specific embodiments of the invention

The following will illustrate the present invention in cooperate with examples, the examples are only used to illustrate the technical scheme for the invention but not limited to the present invention.

### EXAMPLE 1-3:

### The preparation of compounds I, II and III.

### Route A:

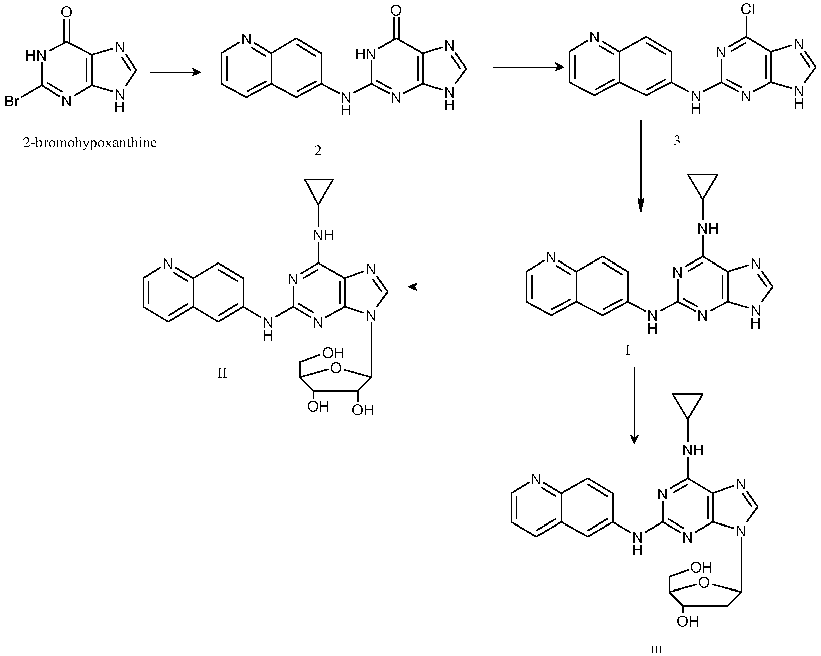

### Example 1. Preparation of Title Compound I

1). In 200ml water, 20g (93mmol) of 2-bromo- hypoxanthine, 13g (90mmol) of 6-aminoquinoline, 60ml ethylene glycol monomethyl ether were mixed and the mixture was refluxed for 48 hours checked the completion of reaction with TLC analysis. The reaction mixture was then poured into ice-water, the solid was isolated by filtration, washed with 200 ml concentrate ammonia water and 3×50 ml methanol, and dried. The crude product obtained was purified by column chromatography on silica gel to afford 14.2 g of compound 2.(yield 57%)
2). 12g (43mmol) of quinoline compound 2, 150m1 phosphorus oxychloride, and 15 ml of N,N-xylidine were mixed and the mixture was refluxed for 30 minutes. The mixture was then cooled to room temperature. The reaction mixture was then poured into 2000 ml ice-water. After 2 hours, the pH of mixture was adjusted to 3 with acetic acid. The yellow solid was isolated by filtration. The crude product obtained was purified by column chromatography on silica gel to afford 11.5g of chloride 3. (yield 90%)
3). 10g (34mmol) of chloride 3, 10 ml (145mmol) of cyclopropylamine, 28 ml (200 mmol) of triethylamine, and 100 ml of DMF were mixed. The mixture was stirred at 100°C for to react 3 hours. Checked the completion of reaction with TLC analysis. The solvent was then distilled off and the residue was dissolved with ethylene glycol dimethyl ether. The mixture was filtered, and the solvent of filtrate was distilled off and the residue obtained was purified by column chromatography on silica gel to afford 7g (22mmol) target compound I (mp>250°C). (yield 65%)

Compound I: ¹H NMR (DMSO-d₆,δ): 0.640-0.642 (2H, s), 0.845-0.860(2H, m), 3.045 (1 H, s), 7.0411-7.432 (1 H, m), 7.632(1 H, s, peak disappeared after added the heavy water), 7.865-7.888 (2H, m), 7.997-8.018 (1 H, m), 8.116-8.136(1 H, m), 8.623-8.682 (2H, m), 9.242 (1 H, s, peak disappeared after added the heavy water), 12.400 (1 H, s, peak disappeared after added the heavy water). MS (ESI): 318 (M+H⁺) 340 (M+Na⁺).

### Example 2. Preparation of Title compound II

10ml of anhydrous acetonitrile, 4.76 g (15mmol) of compound I obtained from example 1, and 6.3 ml (25mmol) of N,O-Bis(trimethylsilyl) acetamide (BSA) were mixed. The mixture was stirred at room temperature for 1 hour. A solution of 1.27g (4mmol) of tetraacetyl ribofuranose dissolved in 10 ml of acetonitrile, and 1.10 ml(16mmol) of TMSTF was then added to the mixture and was refluxed for 5 hours. 1.25 ml (5mmol) of N,O-Bis(trimethylsilyl)acetamide was added to the mixture, and then stirred for 24 hours. Monitored the completion of reaction with TLC analysis. The solvent was distilled off under reduced pressure and the residue was dissolved with 20 ml of methanol. The reaction mixture was passed ammonia gas for 2 hours. The solvent was distilled off under reduced pressure. The residue obtained was purified by column chromatography on silica gel to afford 4.65g of compound II. (yield 72%)

Compound II: MS (ESI): 450 (M+H⁺), 472 (M+Na⁺).

### Example 3. Preparation of Title compound III

10 g (31.5 mmol) of 2-(6-aminoquinolyl)-6- cyclopropyl purine i.e. compound I obtained from example 1, 1.5 g(37.8 mmol) of 60% NaH, and 150 ml of anhydrous acetonitrile were mixed. The mixture was stirred for 30 minutes in an atmosphere of nitrogen. 12 g (31.5mmol) of 3,5-diparatoluensulfonyl-2-deoxy-β-D- ribofuranose-1-chloride was added batchwise to the mixture in 20 minutes. The mixture was reacted at room temperature for 2 hours. The mixture was filtered, and the solvent of the filtrate was distilled off. The oil residue was purified by column chromatography on silica gel to afford 9.8g of 2-(6-aminoquinolyl)-6-cyclopropyl amino-9-(3,5-diparatoluensulfonyl-2-deoxy-β-D- ribofuranose) purine. (yield 47%)

The above product was added to 25 mmol of sodium methoxide and 400 ml of methanol. The mixture was stirred at room temperature for to react 5 hours. The pH of the mixture was adjusted to 7 with acetic acid. The solvent was distilled off and the residue obtained was purified by column chromatography on silica gel to afford 5 g of 2-(6-aminoquinolyl)-6-cyclopropyl amino-9-(2-deoxy-β-D- ribofuranose) purine, i.e., compound III.
(yield 80%)

Compound III: MS (ESI): 434 (M+H⁺), 456(M+Na⁺).

### Example 4: Preparation of compound I Preparation of compound I as described in example 1 according to following Routine B:

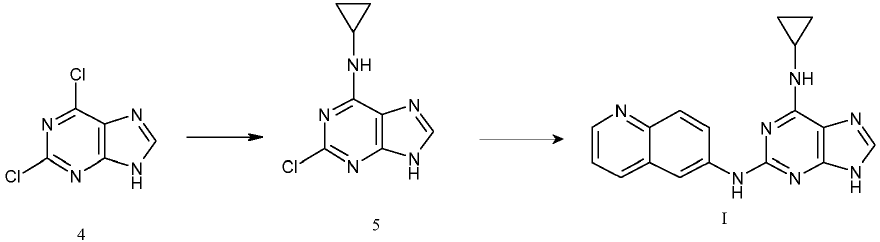

1). 3.78 g (20 mmol) of dichloropurine 4, was dissolved in 50 ml of DMF, 1.4 ml (20mmol) of cyclopropylamine, and 3.08 ml (22mmol) of triethylamine were added. The mixture was reacted at 80°C for 5 hours.
   The completion of reaction was checked with TLC. The solvent was distilled off under reduced pressure and the residue obtained was purified by column chromatography on silica gel to afford 3.34g of cyclopropyl compound 5. (yield 80%)
2). 2.99 g (14.3 mmol) of cyclopropyl compound 5, 5.1 g (36.1 mmol) of 6-amino- quinoline, 50 ml of DMF, and 2.4ml (17.1 mmol) of triethylamine were mixed. The mixture was refluxed at 140°C for 72 hours. TLC analysis proved that the reaction was basically completed. The solvent was distilled off under reduced pressure. The residue obtained was purified by column chromatography on silica gel to afford 3.54 g of compound I. (yield 78%)

Examples 5-7 illustrate the preparation of compounds IV, V, and VI.

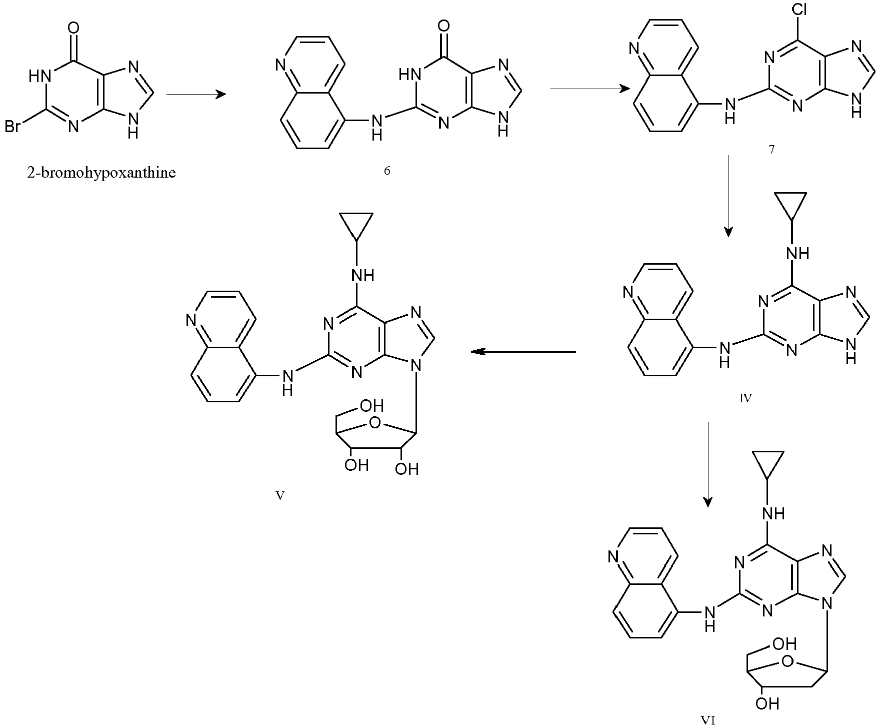

### Example 5: Preparation of Compound IV

1). In 200 ml water, 20 g (93 mmol) of 2-bromo- hypoxanthine, 13g (90 mmol) of 5-aminoquinoline, and 60 ml ethylene glycol monomethyl ether were mixed and the mixture was refluxed for 48 hours. Monitored the completion of reaction with TLC analysis. The reaction mixture was cooled to room temperature then poured into ice-water, and the solid was isolated by filtration, washed with 200 ml concentrate ammonia water and three times of 50 ml methanol, and dried. The residue obtained was purified by column chromatography on silica gel to afford 8g of product 6. (yield 32%)
2). 12 g (43 mmol) of quinoline product 6, 150ml phosphorus oxychloride, and 15ml of N,N-xylidine were mixed and the mixture was refluxed for 30 minutes. The mixture was then cooled at room temperature then poured into 2000 ml ice-water after 2 hours, the pH of mixture was adjusted to 3 with acetic acid. The yellow solid was isolated by filtration. The residue obtained was purified by column chromatography on silica gel to afford 12.0 g of chloride 7. (yield 94%)
3). 10g (34 mmol) of chloride 7, 10 ml (145 mmol) of cyclopropylamine, 28 ml (200 mmol) of triethylamine, and 100 ml of DMF were mixed. The mixture was reacted at 100°C for 3 hours. The completion of reaction was monitored with TLC analysis. The solvent was then distilled off and the residue was dissolved with ethylene glycol dimethyl ether. The mixture was filtered, and the solvent of filtrate was distilled off and the residue obtained was purified by column chromatography on silica gel to afford 9 g compound IV. (yield 84%)

Compound IV: MS (ESI): 318 (M+H⁺), 340(M+Na⁺)

### Example 6: preparation of compound V

10 ml of anhydrous acetonitrile, 4.76 g (15mmol) of compound IV obtained by example 5, and 6.3 ml (25 mmol) of N,O-Bis(trimethylsilyl) acetamide (BSA) were mixed. The mixture was stirred at room temperature for 1 hour. A solution of 1.27g (4mmol) of tetraacetyl ribofuranose dissolved in 10 ml of acetonitrile, and 1.10 ml (16mmol) of TMSTF was then added to the mixture and was refluxed for 5 hours. 1.25 ml (5mmol) of N,O-Bis(trimethylsilyl) acetamide (BSA) was added to the mixture, and then stirred for 24 hours. Monitored the completion of reaction with TLC analysis. The solvent was distilled off in reduced pressure and the residue was dissolved in 20 ml of methanol. The reaction mixture was passed ammonia gas for 2 hours in reduced pressure. The solvent was distilled off. The residue obtained was purified by column chromatography on silica gel to afford 4.07 of compound V.
(yield 63%)

Compound V: MS (ESI): 450 (M+H⁺), 472(M+Na⁺)

### Example 7: Preparation of Compound VI

10g (31.5mmol) of compound IV, i.e. 2-(5-aminoquinolyl)-6- cyclopropyl amino purine, obtained in example 5, 1.5 g(37.8mmol) of 60% NaH, and 150ml of anhydrous acetonitrile were mixed. The mixture was stirred for 30 minutes in an atmosphere of nitrogen. 12 g(31.5mmol) of 3,5-diparatoluensulfonyl-2-deoxy-β-D- ribofuranose-1- chloride was added batchwise to the mixture in 20 minutes. The mixture was reacted at room temperature for 2 hours. The mixture was filtered, and the solvent of the filtrate was distilled off. The oil residue was purified by column chromatography on silica gel to afford 8.0 g of 2-(5-aminoquinolyl)-6-cyclopropyl amino-9-(3,5-diparatoluensulfonyl-2-deoxy-β-D- ribofuranose) purine. (yield 38%)

The above product was added to 25 mmol of sodium methoxide and 400 ml of methanol. The mixture was stirred at room temperature for 5 hours. The pH of mixture was adjusted to 7 with acetic acid. The solvent was distilled off and the residue obtained was purified by column chromatography on silica gel to afford 5.75 g of 2-(5-aminoquinolyl)-6- cyclopropyl amino-9-(2-deoxy-β-D- ribofuranose) purine i.e. compound VI. (yield 92%)

Compound VI: MS (ESI): 434 (M+H⁺), 456 (M+Na⁺)

### Example 8: Preparation of Compound IV, Alternative to Example 5

The process of preparation of Compound IV was as follows: 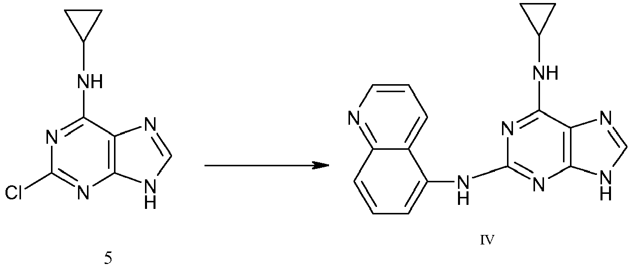 2.99 g (14.3 mmol) of cyclopropyl compound 5, 5.1 g (36.1 mmol) of 5-amino- quinoline, and 50ml of DMF, 2.4ml (17.1 mmol) of triethylamine were mixed. The mixture was refluxed at 140 °C to react for 72 hours. The TLC analysis proved that the reaction was basically completed. The solvent was distilled off under reduced pressure. The residue obtained was purified by column chromatography on silica gel to afford 2.88 g of comound IV. (yield 63%)

### Examples 9-11 illustrate the preparation of compounds VII, VIII and IX.

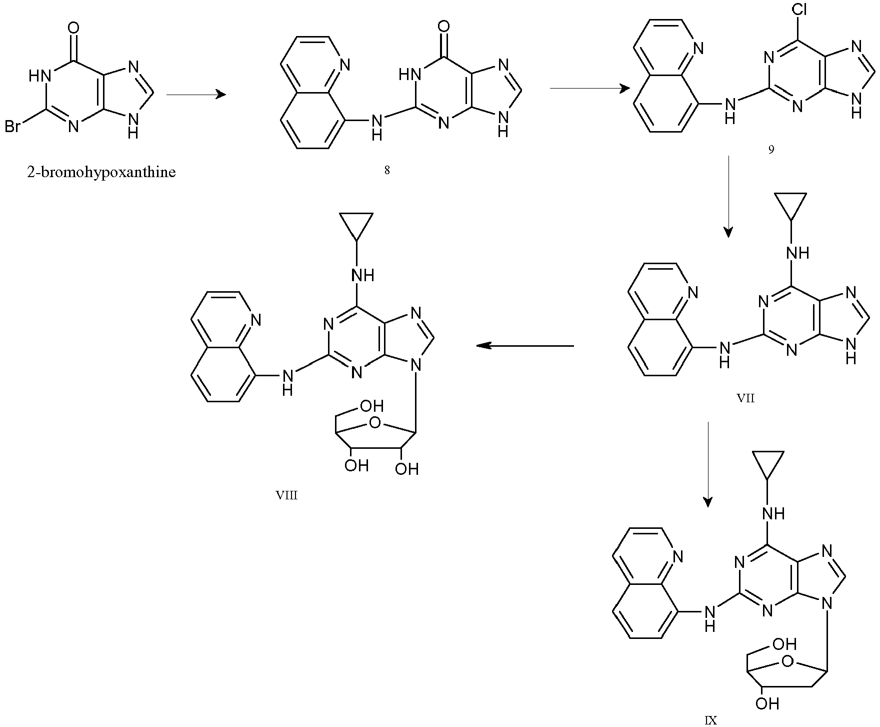

### Example 9: Preparation of Compound VII

1). In 200 ml water, 20 g (93mmol) of 2-bromo- hypoxanthine, 13 g (90 mmol) of 8-aminoquinoline, and 60 ml ethylene glycol monomethyl ether were mixed and the mixture was refluxed for 48 hours. TLC test monitored the completion of reaction. The reaction mixture was then poured into ice-water, the solid was isolated by filtration, washed with 200 ml concentrate ammonia water and 3×50 ml methanol, and dried. The residue obtained was purified by column chromatography on silica gel to afford 11.4 g of product 8.(yield 46%)
2). 12 g (43 mmol) of quinoline product 8, 150 ml of phosphorus oxychloride, and 15 ml of N,N-xylidine were mixed and the mixture was refluxed for 30 minutes. The mixture was then cooled at room temperature then poured into 2000 ml ice-water after 2 hours the pH of mixture was adjusted to 3 with acetic acid. The yellow solid was isolated by filtration. The residue obtained was purified by column chromatography on silica gel to afford 10.3g of chloride 9.(yield 81 %)
3). 10 g (34 mmol) of chloride 9, 10 ml (145 mmol) of cyclopropylamine, 28 ml (200 mmol) of triethylamine, and 100ml of DMF were mixed. The mixture was reacted at 100°C for 3 hours. TLC test monitored the completion of reaction. The solvent was then distilled off and the residue was dissolved with ethylene glycol dimethyl ether. The mixture was filtered, and the solvent of filtrate was distilled off and the residue obtained was purified by column chromatography on silica gel to afford 6 g compound VII.(yield 56%)

Compound VII: MS (ESI) :318 (M+H⁺), 340 (M+Na⁺)

### Example 10: Preparation of Compound VIII

10 ml of anhydrous acetonitrile, 4.76 g (15 mmol) of compound VII, and 6.3 ml (25 mmol) of N,O-Bis(trimethylsilyl) acetamide (BSA) were mixed. The mixture was stirred at room temperature for 1 hour. A solution 1.27 g(4mmol) of tetraacetyl ribofuranose dissolved in 10 ml of acetonitrile, and 1.10 ml(16mmol) of TMSTF was then added to the mixture and was refluxed for 5 hours. 1.25 ml (5 mmol) of N,O-Bis(trimethylsilyl) acetamide was added to the mixture, and then stirred for 24 hours. TLC monitored the completion of reaction. The solvent was distilled off in reduced pressure and the residue was dissolved with 20 ml of methanol. The reaction mixture was in passed ammonia gas for 2 hours. The solvent was distilled off under reduced pressure. The residue obtained was purified by column chromatography on silica gel to afford 4.2 g of compound VIII.(yield 65%)

Compound VIII: MS (ESI): 450 (M+H⁺), 472(M+Na⁺).

### Example 11: Preparation of Compound IX

10 g (31.5 mmol) of compound VII i.e. 2-(8-aminoquinolyl)-6-cyclopropyl amino purine, 1.5 g(37.8mmol) of 60% NaH, and 150 ml of anhydrous acetonitrile were mixed. The mixture was stirred for 30 minutes in an atmosphere of nitrogen. 12 g(31.5mmol) of 3,5-diparatoluensulfonyl-2-deoxy-β-D- ribofuranose-1- chloride was added batchwise to the mixture in 20 minutes. The mixture was stirred at room temperature to react for 2 hours. The mixture was filtered, and the solvent of the filtrate was distilled off. The oil residue was purified by column chromatography on silica gel to afford 7.4 g of 2-(8-aminoquinolyl)-6- cyclopropyl amino-9-(3,5-diparatoluensulfonyl-2-deoxy-β-D- ribofuranose) purine.(yield 35%)

The above product was added to 25 mmol of sodium methoxide and 400 ml of methanol. The mixture was stirred at room temperature to react for 5 hours. The pH of mixture was adjusted to 7 with acetic acid. The solvent was distilled off and the residue obtained was purified by column chromatography on silica gel to afford 3.2g of 2-(8-aminoquinolyl)-6-cyclopropyl amino-9-(2-deoxy-β-D- ribofuranose) purine compound IX.(yield 51 %)

Compound IX: MS (ESI): 434 (M+H⁺), 456 (M+Na⁺).

### Example 12: Preparation of Compound VII; Alternative to Example 9

The process of preparation of compound was as follows: 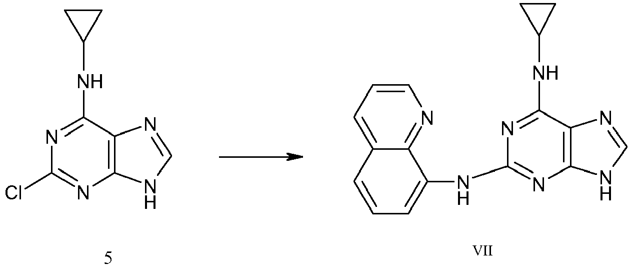 2.99 (14.3 mmol) of cyclopropyl compound 5, 51 g (36.1 mmol) of 8-amino- quinoline, 50 ml of DMF, and 2.4 ml(17.1 mmol) of triethylamine were mixed. The mixture was refluxed at 140°C to react for 72 hours. Check the completion of reaction by TLC analysis. The solvent was distilled off under reduced pressure. The residue obtained was purified by column chromatography on silica gel to afford 4.10 g of compound VII.(yield 90%)

### Examples 13-15 illustrate the preparation of compounds X, XI and XII.

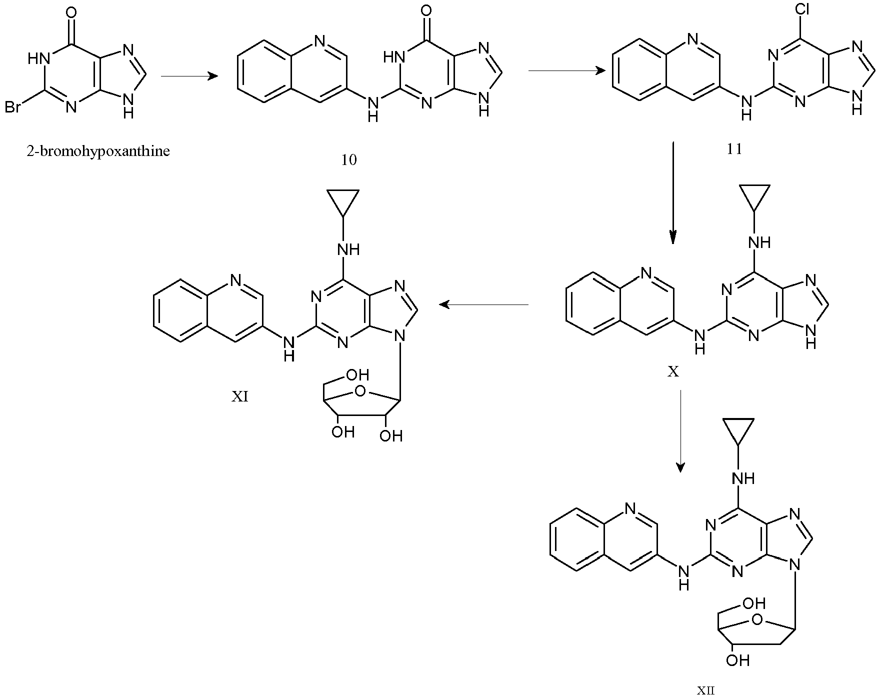

### Example 13: Preparation of Compound X

1). In 200 ml water, 20 g (93 mmol) of 2-bromo- hypoxanthine, 13 g (90 mmol) of 3-aminoquinoline, and 60 ml ethylene glycol monomethyl ether were mixed and the mixture was refluxed for 48 hours. Check the completion of reaction by TLC analysis. The reaction mixture was then poured into ice-water, the solid was isolated by filtration, washed with 200 ml concentrate ammonia water and 3×50 ml methanol, and dried. The residue obtained was purified by column chromatography on silica gel to afford 15.0 g of product 10. (yield 60%)
2). 12g (43 mmol) of compound quinoline product 10, 150 ml phosphorus oxychloride, and 15ml of N,N-xylidine were mixed and the mixture was refluxed for 30 minutes. The mixture was then cooled at room temperature . The reaction mixture was then poured slowly into 2,000 ml ice-water, after 2 hours the pH of mixture was adjusted to 3 with acetic acid. The yellow solid was isolated by filtration. The residue obtained was purified by column chromatography on silica gel to afford 10.9 g of chloride 11. (yield 85%)
3). 10 g (34 mmol) of chloride 11, 10 ml (145 mmol) of cyclopropylamine, 28 ml (200 mmol) of triethylamine, 100 ml of DMF were mixed. The mixture was stirred at 100°C to react for 3 hours, check the completion of reaction by TLC analysis. The solvent was then distilled off and the residue was dissolved with ethylene glycol dimethyl ether. The mixture was filtered, and the solvent of filtrate was distilled off and the residue obtained was purified by column chromatography on silica gel to afford 10.1g compound X (yield 94%).

Compound X: MS (ESI): 318 (M+H⁺), 340 (M+Na⁺).

### Example 14: Preparation of Compound XI

10 ml of anhydrous acetonitrile, 4.76 g (15 mmol) of compound X, and 6.3 ml (25 mmol) of N,O-Bis(trimethylsilyl) acetamide (BSA) were mixed. The mixture was stirred at room temperature for 1 hour, a solution of 1.27 g(4mmol) of tetraacetyl ribofuranose dissolved in 10 ml of acetonitrile, and 1.10 ml(16mmol) of TMSTF was then added to the mixture and was refluxed for 5 hours. 1.25 ml (5 mmol) of N,O-Bis(trimethylsilyl) acetamide was added to the mixture, and then stirred for 24 hours, check the completion of reaction by TLC analysis. The solvent was distilled off under reduced pressure and the residue was dissolved with 20 ml of methanol. The reaction mixture was passed ammonia gas for 2 hours. The solvent was distilled off under reduced pressure. The residue obtained was purified by column chromatography on silica gel to afford 4.97g of compound XI.(yield 77%)

Compound XI: MS (ESI): 450 (M+H⁺), 472 (M+Na⁺).

### Example 15: Preparation of Compound XII

10 g (31.5 mmol) of 2-(3-aminoquinolyl)-6- cyclopropyl amino purine i.e. compound X, 1.5 g(37.8mmol) of 60 % NaH, and 150 ml of anhydrous acetonitrile were mixed. The mixture was stirred for 30 minutes in an atmosphere of nitrogen. 12 g(31.5mmol) of 3,5-diparatoluensulfonyl-2-deoxy-β-D- ribofuranose-1- chloride was added batchwise to the mixture in 20 minutes. The mixture was stirred at room temperature for 2 hours. The mixture was filtered, and the solvent of the filtrate was distilled off. The oil residue was purified by column chromatography on silica gel to afford 8.8g of 2-(3-aminoquinolyl)-6- cyclopropyl amino-9-(3,5-diparatoluensulfonyl-2-deoxy-β-D- ribofuranose) purine.(yield 42%)

The above product was added to 25 mmol of sodium methoxide and 400 ml of methanol. The mixture was stirred at room temperature for 5 hours. The pH of mixture was adjusted to 7 with acetic acid. The solvent was distilled off and the residue obtained was purified by column chromatography on silica gel to afford 5.06g of 2-(3-aminoquinolyl)-6- cyclopropyl amino-9-(2-deoxy-β-D- ribofuranose) purine i.e. compound XII.(yield 81 %)

Compound XII: MS (ESI): 434 (M+H⁺), 456 (M+Na⁺).

### Example 16: Preparation of Compound X, Alternative to Example 9

The process of preparation of compound was as follows: 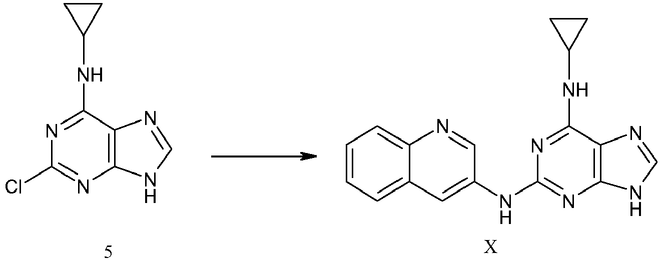 2.99 g (14.3 mmol) of cyclopropyl 5, 51 g (36.1 mmol) of 3-amino-quinoline, 50ml of DMF, and 2.4ml(17.1mmol) of triethylamine were mixed. The mixture was refluxed at 140°C to react for 72 hours. The completion of reaction was monitored by TLC analysis. The solvent was distilled off. The residue obtained was purified by column chromatography on silica gel to afford 3.54 g of compound X.(yield 78%)

### Examples 17-19 illustrate the preparation of compounds VII, XIV, and XV.

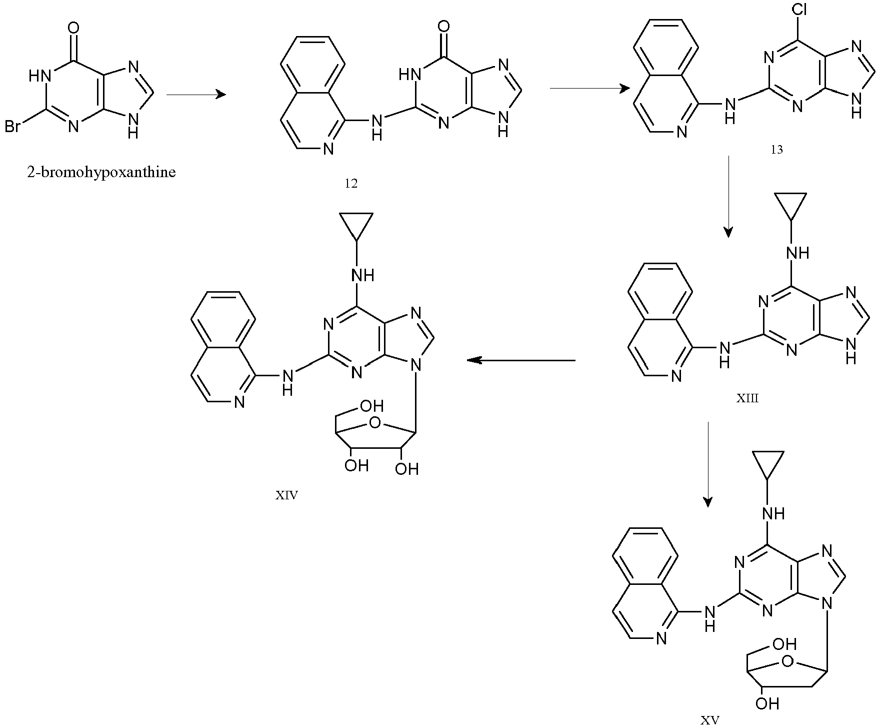

### Example 17: Preparation of Compound XIII

1). In 200 ml water, 20 g (93 mmol) of 2-bromo-hypoxanthine, 13g (90 mmol) of 1-aminoisoquinoline, and 60 ml ethylene glycol monomethyl ether were mixed and the mixture was refluxed for 48 hours. The completion of reaction was monitored by TLC analysis. The reaction mixture was then poured into ice-water, the solid was isolated by filtration, washed with 200 ml concentrate ammonia water and 3×50 ml methanol, dried. The residue obtained was purified by column chromatography on silica gel to afford 14.2g of product 12.(yield 57%)
2). 12 g (43 mmol) of quinoline product 12, 150 ml phosphorus oxychloride, and 15 ml of N,N-xylidine were mixed and the mixture was refluxed for 30 minutes. The mixture was then cooled to room temperature. The reaction mixture was then poured into 2000 ml ice-water after 2 hours the pH of mixture was adjusted to 3 with acetic acid. The yellow solid was isolated by filtration. The residue obtained was purified by column chromatography on silica gel to afford 11.5g of chloride 13. (yield 90%)
3). 10 g (34 mmol) of chloride 13, 10 ml (145 mmol) of cyclopropylamine, 28 ml (200 mmol) of triethylamine, and 100 ml of DMF were mixed. The mixture was stirred at 100°C for 3 hours. The completion of reaction was monitored with TLC analysis. The solvent was then distilled off and the residue was dissolved with ethylene glycol dimethyl ether. The mixture was filtered, and the solvent of filtrate was distilled off and the residue obtained was purified by column chromatography on silica gel to afford 4.2 g compound XIII.(yield 39%)

Compound XIII: MS (ESI): 318 (M+H⁺), 340 (M+Na⁺).

### Example 18: Preparation of Compound XIV

10 ml of anhydrous acetonitrile, 4.76 g (15 mmol) of compound XIII, and 6.3 ml (25 mmol) of N,O-Bis(trimethylsilyl) acetamide (BSA) were mixed. The mixture was stirred at room temperature for 1 hour. A solution of 1.27 g(4mmol) of tetraacetyl ribofuranose dissolved in 10 ml of acetonitrile, and 1.10m1(16mmol) of TMSTF was then added to the mixture and was refluxed for 5 hours. 1.25 ml (5 mmol) of N,O-Bis(trimethylsilyl) acetamide was added to the mixture, and then stirred for 24 hours. The completion of reaction was monitored by TLC analysis. The solvent was distilled off under reduced pressure and the residue was dissolved with 20 ml of methanol. The reaction mixture passed ammonia gas for 2 hours. The solvent was distilled off under reduced pressure. The residue obtained was purified by column chromatography on silica gel to afford 4.2g of compound XIV. (yield 64%)

Compound XIV: MS (ESI): 450 (M+H⁺), 472 (M+Na⁺).

### Example 19: Preparation of Compound XV

10 g (31.5 mmol) of compound XIII i.e. 2-(1-aminoquinolyl)-6-cyclopropyl amino purine., 1.5 g (37.8mmol) of 60% NaH, and 150 ml of anhydrous acetonitrile were mixed. The mixture was stirred for 30 minutes in an atmosphere of nitrogen. 12 g(31.5mmol) of 3,5-diparatoluensulfonyl-2-deoxy-β-D- ribofuranose-1- chloride was added batchwise to the mixture in 20 minutes. The mixture was stirred at room temperature to react for 2 hours. The mixture was filtered, and the solvent of the filtrate was distilled off. The oil residue was purified by column chromatography on silica gel to afford 7.09g of 2-(1-aminoisoquinolyl)-6- cyclopropyl amino-9-(3,5-diparatoluensulfonyl-2-deoxy-β-D- ribofuranose) purine.(yield 34%)

The above product was added to 25mmol of sodium methoxide and 400ml of methanol. The mixture was stirred at room temperature to react for 5 hours. The pH of mixture was adjusted to 7 with acetic acid. The solvent was distilled off and the residue obtained was purified by column chromatography on silica gel to afford 3.8 g of compound XV. (yield 60.8%)

Compound XV: MS (ESI): 434 (M+H⁺), 456 (M+Na⁺).

### Example 20: Preparation of Compound XIII, Alternative to Example 17

The process of preparation of compound XIII was as follows:

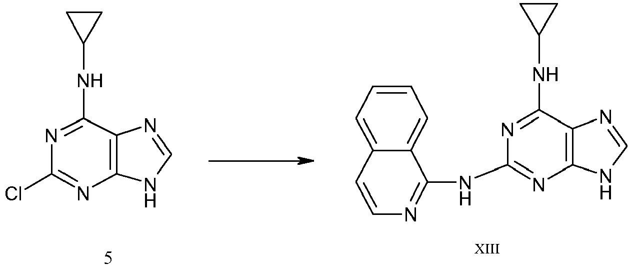

2.99 g (14.3 mmol) of cyclopropyl 5, 51 g (36.1 mmol) of 3-amino-quinoline, 50ml of DMF, and 2.4ml(17.1 mmol) of triethylamine were mixed. The mixture was refluxed at 140°C to react for 72 hours. The completion of reaction was monitored by TLC analysis. The solvent was distilled off. The residue obtained was purified by column chromatography on silica gel to afford 4.16g of compound XIII. (yield 92%)

### Examples 21-22 illustrate the preparation of compound XVI, XVII, and XVIII.

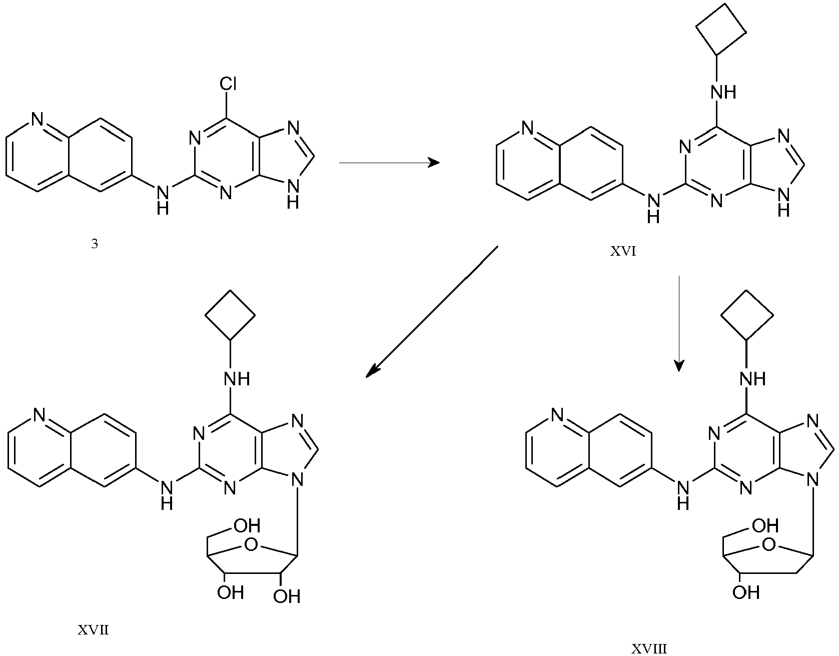

### Example 21: Preparation of Compound XVI

10 g (34 mmol) of compound 3, 10.3g(145mmol) of cyclobutylamine, 28 ml (200 mmol) of triethylamine, and 100 ml of DMF were mixed. The mixture was reacted at 100°C for 3 hours. The completion of reaction was checked by TLC analysis. The solvent was then distilled off and the residue was dissolved with ethylene glycol dimethyl ether. The mixture was filtered, and the solvent of filtrate was distilled off and the residue obtained was purified by column chromatography on silica gel to afford 6.8g compound XVI.
(yield 60%)

Compound XVI: MS (ESI): 332 (M+H⁺), 354 (M+Na⁺).

### Example 22: Preparation of Compound XVII

10 ml of anhydrous acetonitrile, 4.97 g(15mmol) of compound XVI, and 6.3 ml (25 mmol) of N,O-Bis(trimethylsilyl)acetamide (BSA) were mixed. The mixture was stirred at room temperature for 1 hour. A solution of 1.27 g (4mmol) of tetraacetyl ribofuranose dissolved in 10 ml of acetonitrile, and 1.10 ml(16mmol) of TMSTF were then added to the mixture and refluxed for 5 hours. 1.25 ml (5 mmol) of N,O-Bis(trimethylsilyl)acetamide was added to the mixture, and then stirred for 24 hours. Check the completion of reaction by TLC analysis. The solvent was distilled off under reduced pressure and the residue was dissolved in 20 ml of methanol. The reaction mixture passed ammonia gas for 2 hours. The solvent was distilled off under reduced pressure. The residue obtained was purified column chromatography on silica gel to afford 4.71 g of compound XVII. (yield 71 %)

Compound XVII: MS (ESI): 464 (M+H⁺), 486 (M+Na⁺).

### Example 23: Preparation of Compound XVIII

10 g (31.5mmol) of compound XVI,i.e. 2-(6-aminoquinolyl)-6- cyclobutyl amino purine. 1.5 g(37.8mmol) of 60% NaH, and 150 ml of anhydrous acetonitrile were mixed. The mixture was stirred for 30 minutes in an atmosphere of nitrogen. 12 g(31.5mmol) of 3,5-diparatoluensulfonyl-2-deoxy-β-D- ribofuranose-1- chloride was added batchwise to the mixture in 20 minutes. The mixture was stirred at room temperature to react for 2 hours. The mixture was filtered, and the solvent of the filtrate was distilled off. The oil residue was purified by column chromatography on silica gel to afford 2-(6-aminoquinolyl)-6- cyclobutyl amino-9-(3,5-diparatoluensulfonyl-2-deoxy-β-D-ribofuranose) purine.

The above product was added to 25 mmol of sodium methoxide and 400 ml of methanol. The mixture was stirred at room temperature to react for 5 hours. The pH of mixture was adjusted to 7 with acetic acid. The solvent was distilled off and the residue obtained was purified by column chromatography on silica gel to afford 7 g of compound XVIII.i.e. 2-(6-aminoquinolyl)-6- cyclobutyl amino-9-(2-deoxy-β-D- ribofuranose) purine.
(yield 51.8%)

Compound XVIII: MS (ESI): 448 (M+H⁺), 470 (M+Na⁺).

### Example 24: Preparation of Compound XVI

The process of preparation of compound was as follows: 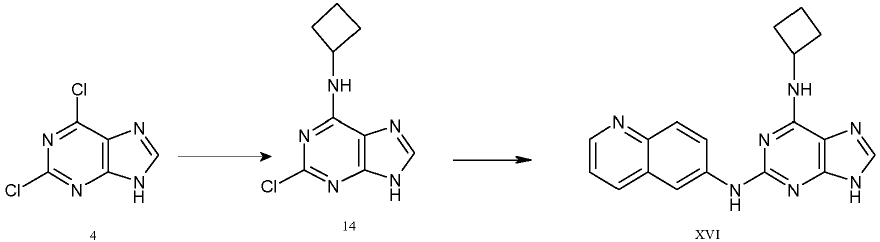
1). 3.78 g (20 mmol) of dichloropurine 4, was dissolved in 50 ml of DMF, 1.4 ml(20mmol) of cyclobutylamine, and 3.08 ml(22mmol) of triethylamine were mixed. The mixture was stirred at 80 °C to react for 5 hours. The completion of reaction was monitored by TLC analysis. The solvent was distilled off under reduced pressure and the residue obtained was purified by column chromatography on silica gel to afford 4.1 g of cyclobutyl compound 14. (yield 92%)
2). 3.19 g (14.3 mmol) of cyclobutyl compound 14, 5.1 g (36.1 mmol) of 6-amino- quinoline, 50 ml of DMF, and 2.4 ml(17.1 mmol) of triethylamine were mixed. The mixture was refluxed at 140°C for 72 hours. The reaction was basically completed by TLC checking. The solvent was distilled off under reduced pressure. The residue obtained was purified by column chromatography on silica gel to afford 3.82g of compound XVI. (yield 81 %)

### Example 25: Preparation of Hydrochloride and Lactate of Compound I

### Preparation of the hydrochloride of compound I:

10 g(31.54mmol) of compound I, 210 ml of ethanol, and 25 ml of water were mixed. The mixture was heated to dissolve compound I. 0.7 ml(37.85mmol) of 2 mol/L HCl was added dropwise to the mixture. The mixture was refluxed for 0.5 hour. The mixture was allowed to cool slowly at room temperature, and then was allowed to cool to below 5°C and stand for 5 hours. The pale yellow solid was isolated by filtration. The pale yellow solid was 10g after dried. (yield 90%) The melting point of the pale yellow solid was above 270°C.

### Preparation of the lactate of compound I:

5g of compound I,(15.77mmol) 105 ml of 95% ethanol, and 12.5 ml of H₂O were mixed. The mixture was heated to dissolve compound I. 10% lactic acid in ethanol solution was added dropwise to the mixture. The mixture was then refluxed for 1 hour. The mixture was allowed to cool to room temperature, and then was allowed to cool to below 5°C for 5 hours. The pale yellow solid was isolated by filtration. The pale yellow solid was 5.6g after dried.(yield 87%) The melting point of the pale yellow solid was 239-248°C.

### Example 26: Preparation of the Pharmaceutical Dosage Forms

### Preparation of coated tablets:

Formula of the core of the tablets:

| | |
|---|---|
| Compound 1 | 50 g |
| microcrystalline cellulose | 150 g |
| lactose | 50 g |
| Sodium Carboxy Methyl Starch Soluble | 25 g |
| Sodium Cros Carboxy Methyl Cellulose | 15 g |
| Micro Powder Silica Gel | 1.5 g |
| For | 1000 Tablets |

The process: the exact weight of compound I and lactose was mixed, then silica gel was added to the mixture in order to increase the fluidity. Other pharmaceutical adjuvants were added and mixed, followed by direct tabletting.

Formula of the coating liquid: Opadry 25 g, in a suitable amount of 80% ethanol, for coating.

### Preparation of Injection:

### Formula of the injection:

| | |
|---|---|
| Compound I | 50 g |
| Tween-80 | 20 g |
| Ethyl alcohol for pharmaceutical use | 30 g |
| Water for injection | added to 10,000 mL |
| For | 1000 vials |

The process: the exact weight of compound I and Tween-80 was mixed and triturated, 0.3% ethyl alcohol was added to the mixture to dissolve compound I and Tween-80 with heating. The liquid was filtered with 0.22 µm filter membrane under aseptic conditions, and then filled 10 ml per vial, and steam sterilized at 100°C.

## Claims

1. A compound having the following formula A, its salts, or hydrates: 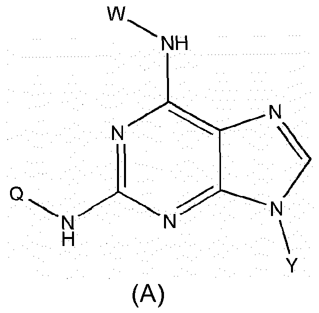 wherein
W represents a substituent having any one of the following formulas: 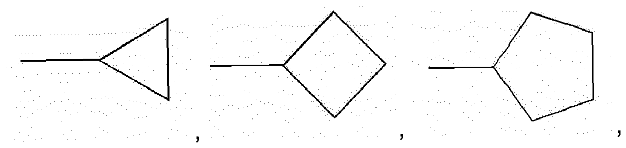 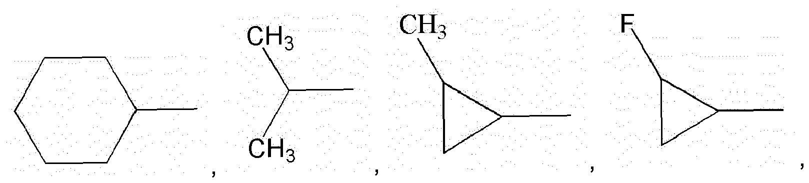 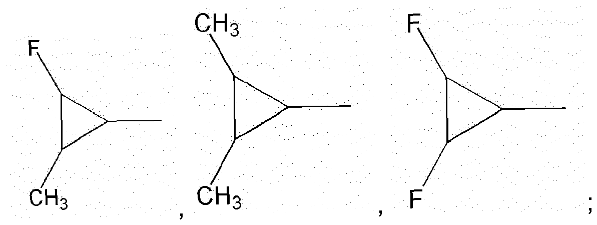
Y represents hydrogen, or a pharmaceutically-acceptable saccharide of any one of the following formulas: 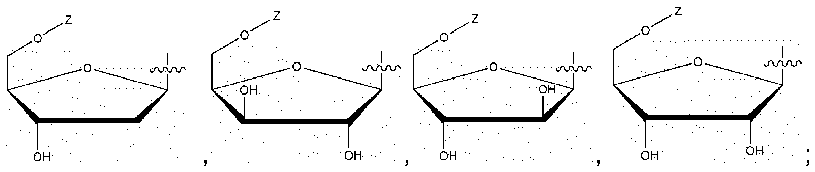
Z represents hydrogen or a substituent having any one of the following formulas: 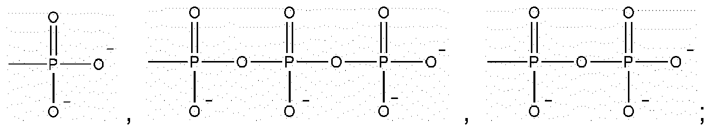
Q represents a substituent having any one of the following formulas: 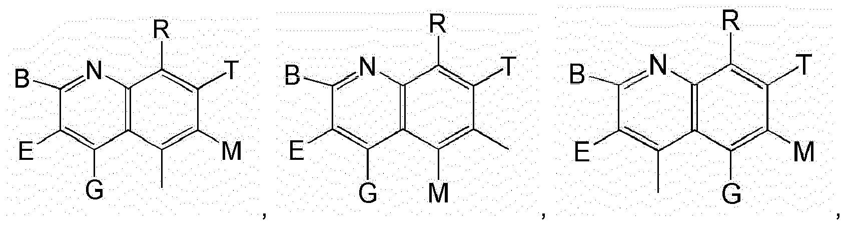 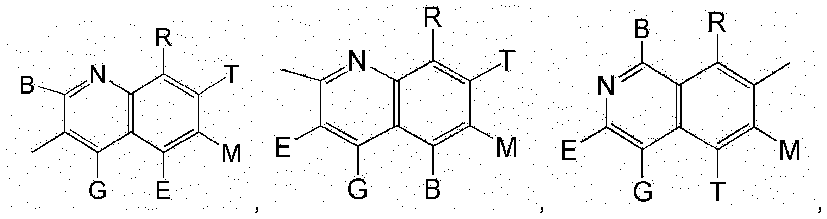 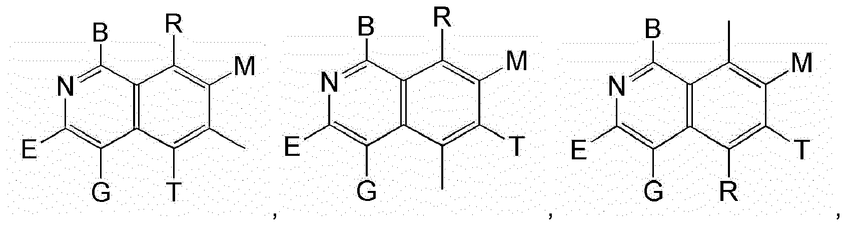 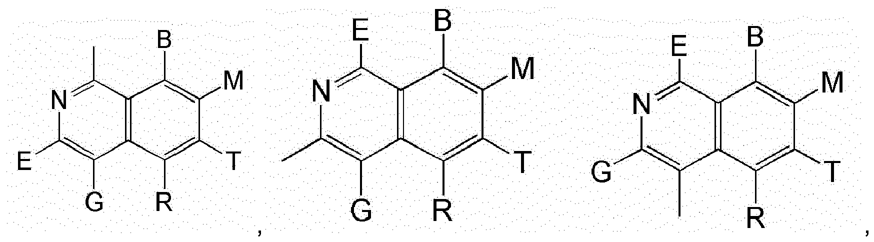 and
B, E, G, R, T, and M each independently represent hydrogen, a straight or branched C₁₋₆ alkyl or haloalkyl, a C₃₋₆ cycloalkyl, a halogen, a cyano, or an amino group.

2. The compound of formula (A), its salts, or hydrates according to claim 1, wherein Y represents hydrogen.

3. The compound of formula (A), its salts, or hydrates according to claim 1,
wherein
W represents a substituent having any one of the following formulas: 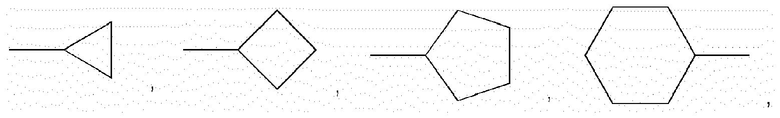 and
B, E, G, R, T, and M independently and at each occurrence represent hydrogen.

4. The compound of formula (A), its salts, or hydrates according to claim 1 or 2, wherein
Wis 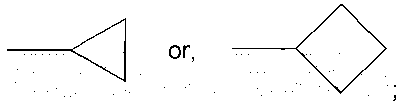
Q is a substituent having any one of the following formulas:

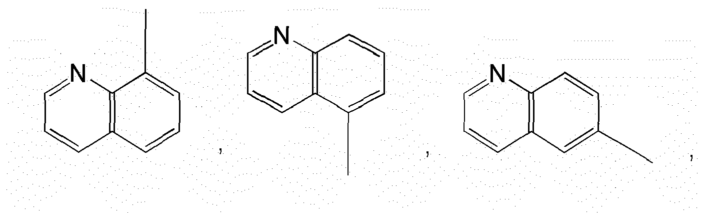

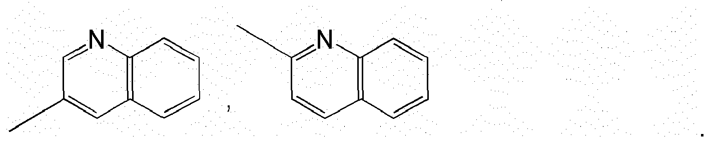

5. The compound of formula (A), its salts, or hydrates according to claim 4, wherein Q represents

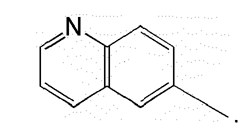

6. The compound of formula (A), its salts, or hydrates according to claim 1, wherein the compound is represented by any one of the following formulas (I-XVIII):

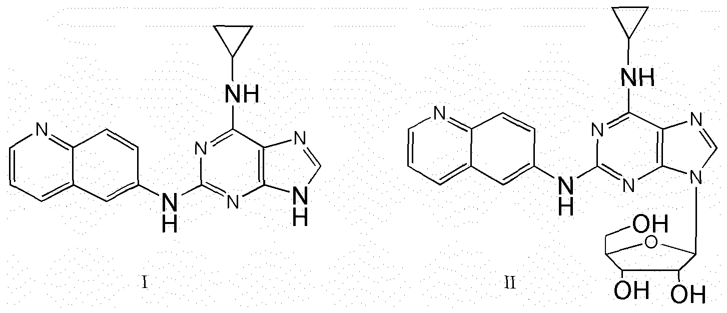

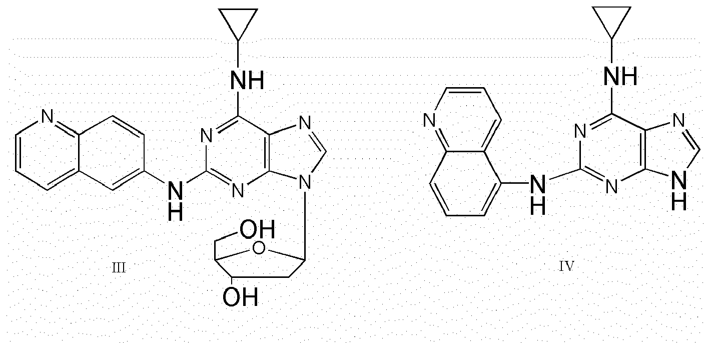

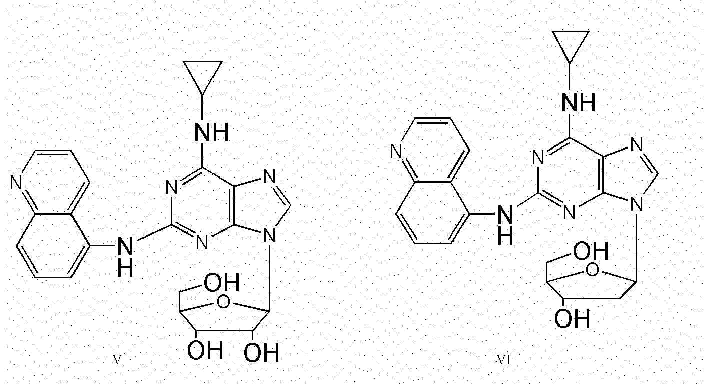

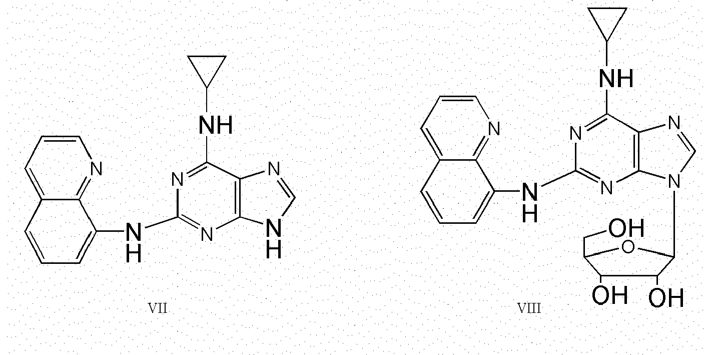

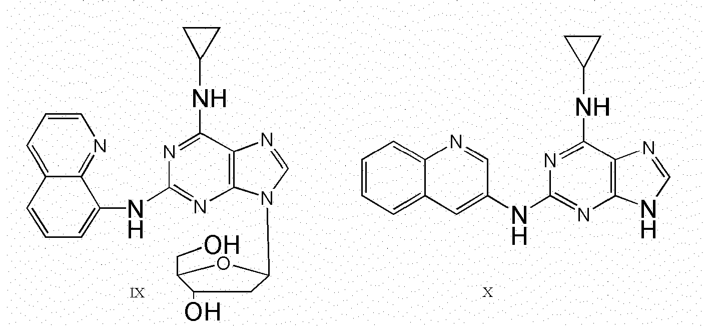

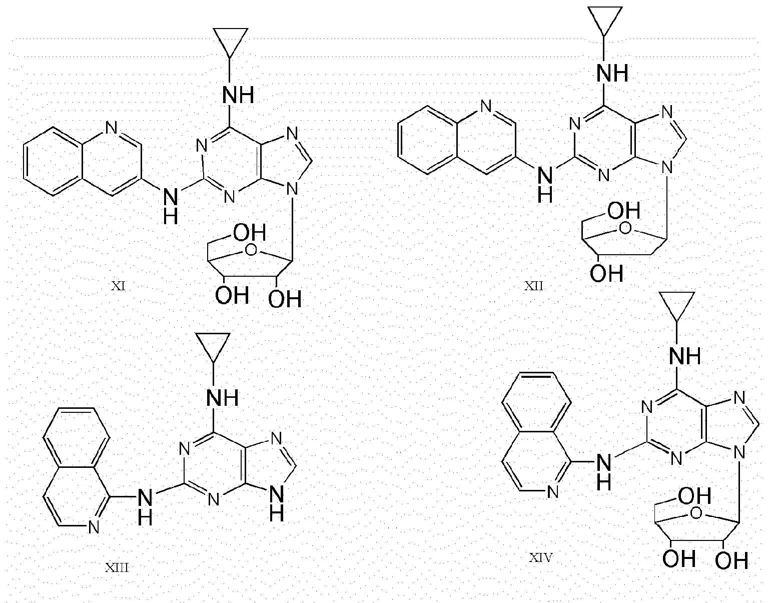

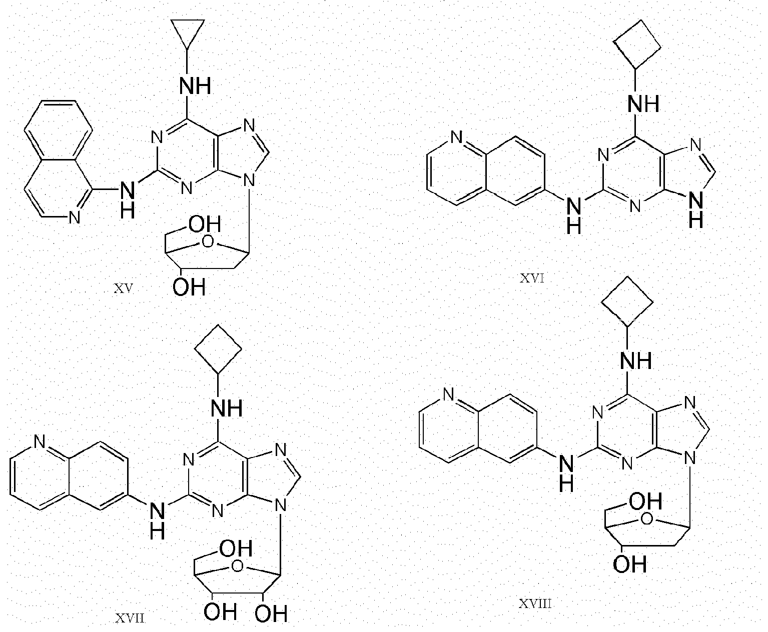

7. The compound of formula (A), its salts, or hydrates according to claim 4, wherein the compound is represented by the following formula (I):

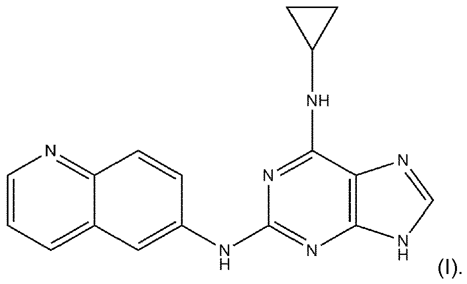

8. A pharmaceutical composition comprising the compound of formula (A), its salts, or hydrates according to any one of claims 1 to 7, and a pharmaceutically acceptable excipient.

9. The pharmaceutical composition according to claim 8, wherein the described pharmaceutical compositions is in the form of a tablet, a capsule, a pill, an oral liquid preparation, a granule, a powder, an injection, an implant, or a preparation for external use.

10. A method for producing the compound of formula (A), its salts, or hydrates according to any one of claims 1 to 7, the method comprising following steps:
(1) reacting the compound (j) with 0.8-1.5 mol/mol Q-NH₂ in an organic solvent at a temperature of about 50 to about 150°C for 1-72 hours, to result in a first reaction mixture, followed by adding water to the first reaction mixture, and cooling the first reaction mixture at room temperature, to produce a compound of formula (b); 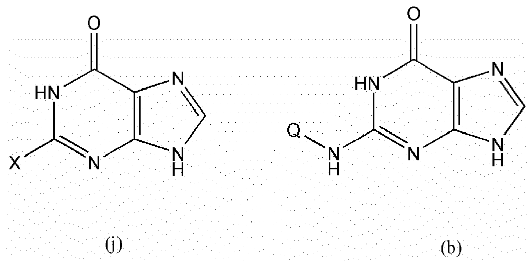
(2) reacting the compound of formula (b) with a halogenating agent in an organic solvent at about 50 to about 150 °C for 1-72 hours, cooling, followed by adding water to the reaction mixture and adjusting the pH of the reaction mixture to about 2-5 with an acid, and allowing the second reaction mixture to cool at a room temperature, to produce a compound of the formula (c): 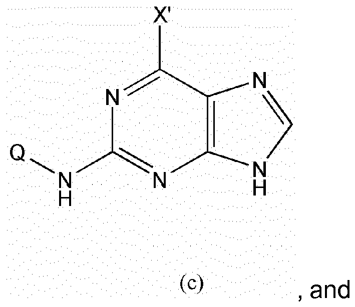
(3) reacting compound (c) in an organic solvent with 0.8-1.5 mol/mol of W-NH₂ in the presence of an acid acceptor at a temperature of about 50-150°C for 1-72 hours, followed by distilling off the solvent; to yield a compound of formula (f), 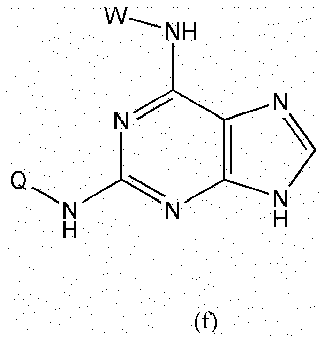 wherein X represents Br, X' represents Cl, and W and Q are as defined in claim 1.

11. A method for producing the compound of formula (A), its salts, or hydrates according to any one of claims 1 to 7, the method comprising the following steps:
(1) reacting in an organic solvent, the compound of formula (k) with 0.8-1.5 mol/mol of W-NH₂, in the presence of an acid acceptor at a temperature of about 30-120°C for 1-72 hours, followed by distilling off the solvent, to produce a compound of formula (e): 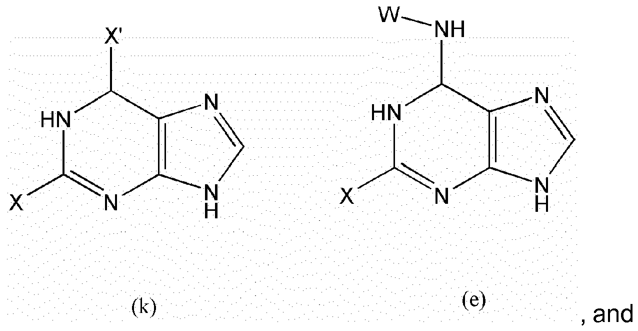
(2) reacting in an organic solvent the compound of formula (e) with 0.8-1.5 mol/mol of Q-NH₂, in the presence of an acid acceptor at a temperature of about 70-170°C for 1-72 hours, followed by distilling off the solvent to produce a compound of formula (f): 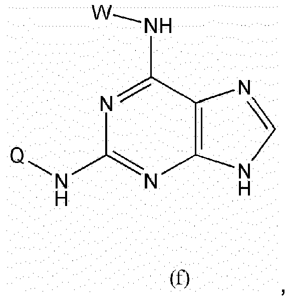 wherein X represents Cl, X' represents Cl, and W and Q are as defined in claim1.

12. The compound according to claim 1, wherein the salts are pharmaceutically acceptable salts, which can be addition salts, produced by organic or inorganic acids, and are preferably hydrochloride salts, hydrobromide salts, hydroiodide salts, p-toluenesulfonate salts, phosphate salts, sulfate salts, perchloride salts, acetate salts, trifluoroacetate salts, propionate salts, citrate, malonate salts, succinate, lactate salts, oxalate, tartrate salts and benzoate salts, the salt also can be formed with a base, including an inorganic or organic base.

13. The use of any one of the compounds, their salts, or hydrates according to claims 1-7 in the preparation of pharmaceuticals for prevention and treatment of tumor diseases.

14. The use of any one of the compounds, their salts, or hydrates according to claims 1-7 in the preparation of pharmaceuticals for prevention and treatment of tumor diseases, wherein said tumor diseases are lung cancer, liver cancer or, leukemia.

## Patentansprüche

1. Verbindung der Formel A, ihre Salze, oder Hydrate: 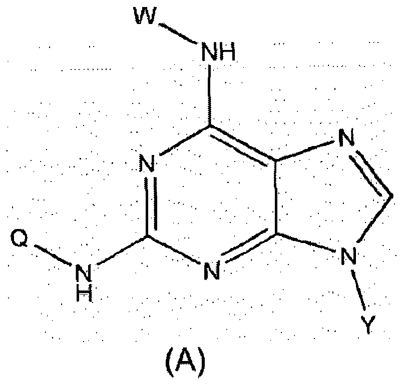 wobei
W ein Substituent mit einer der nachfolgenden Formeln ist: 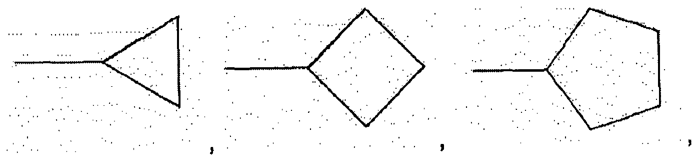 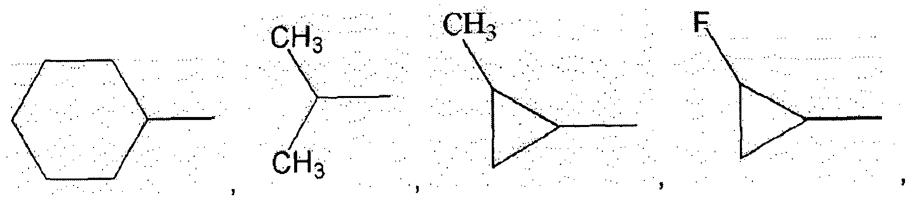 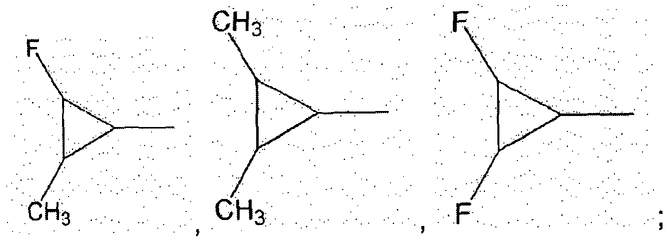
Y Wasserstoff, oder ein pharmazeutisch-akzeptables Saccharid nach einer der folgenden Formeln ist: 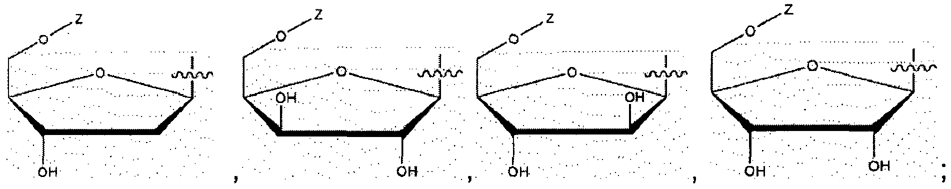
Z Wasserstoff, oder ein Substituent nach einer der folgenden Formeln ist: 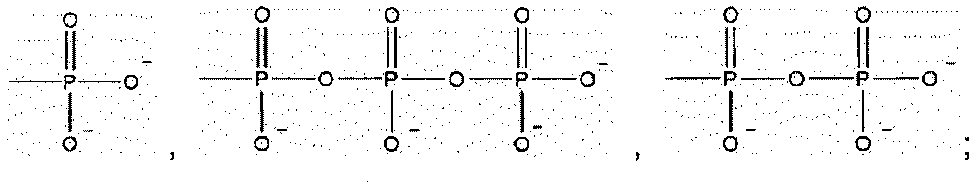
Q ein Substituent nach einer der folgenden Formeln ist: 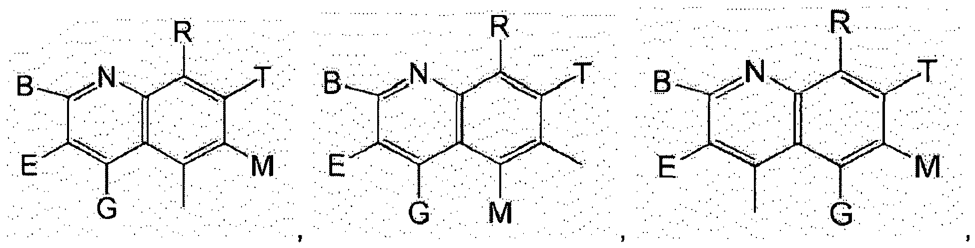 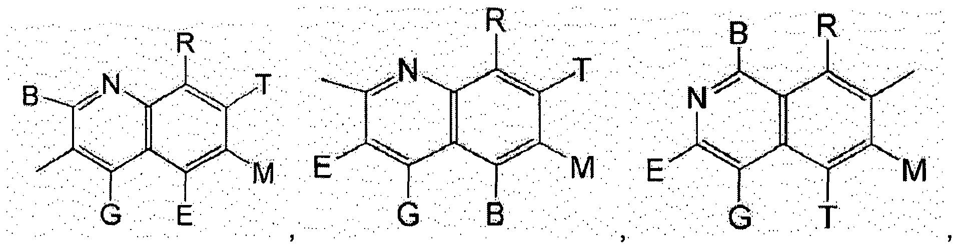 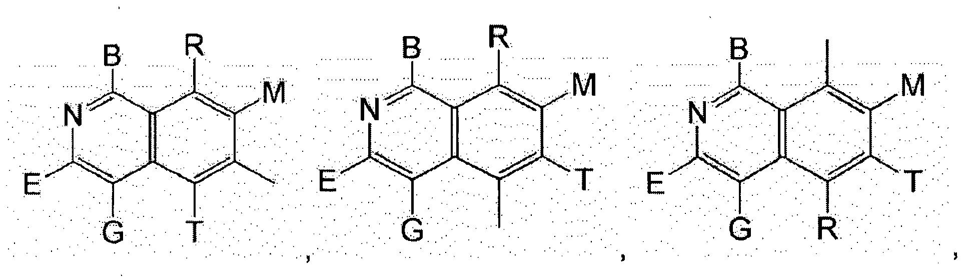 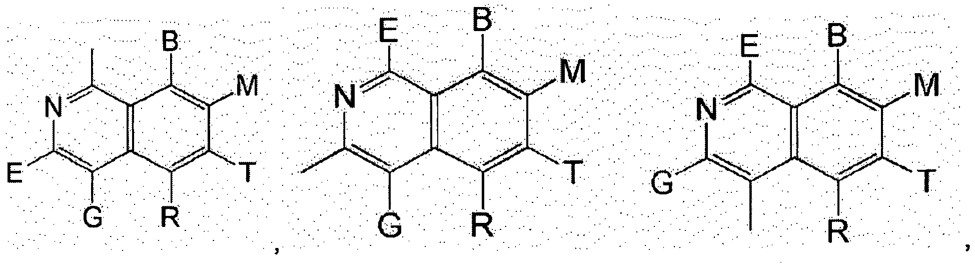 und
B, E, G, R, T, und M jeweils unabhängig Wasserstoff, ein alipathisches oder verzweigtes C₁₋₆-Alkyl oder Halogenalkyl, ein C₃₋₆-Cycloalkyl, eine Halogengruppe, eine Cyanogruppe oder eine Aminogruppe ist.

2. Verbindung der Formel (A), ihre Salze, oder Hydrate nach Anspruch 1, wobei Y ein Wasserstoff ist.

3. Verbindung der Formel (A), ihre Salze, oder Hydrate nach Anspruch 1, wobei
W ein Substituent nach einer der folgenden Formeln ist: 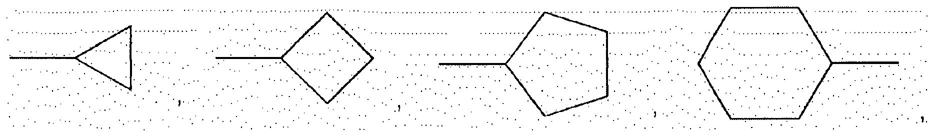 und
B, E, G, R, T, und M jeweils an jeder Stelle Wasserstoff sind.

4. Verbindung der Formel (A), ihre Salze, oder Hydrate nach Anspruch 1 oder 2, wobei
W ein Substituent nach einer der folgenden Formeln ist: 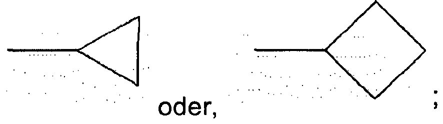
Q ein Substituent nach einer der folgenden Formeln ist:

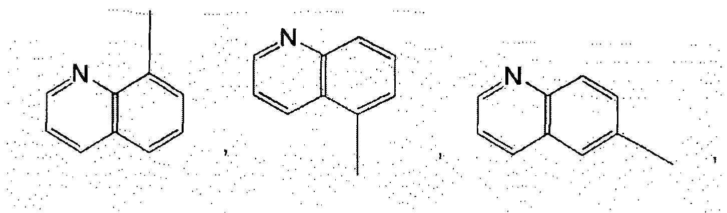

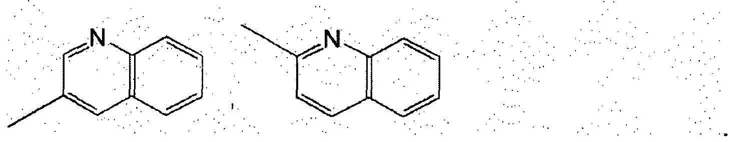

5. Verbindung der Formel (A), ihre Salze, oder Hydrate nach Anspruch 4, wobei Q

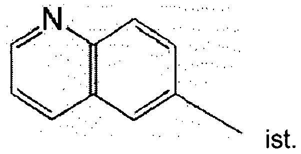

6. Verbindung der Formel (A), ihre Salze, oder Hydrate nach Anspruch 1, wobei die Verbindung durch eine der nachfolgenden Formeln (I - XVIII) dargestellt ist:

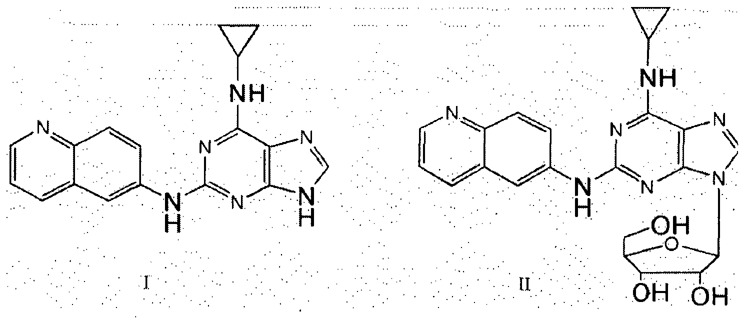

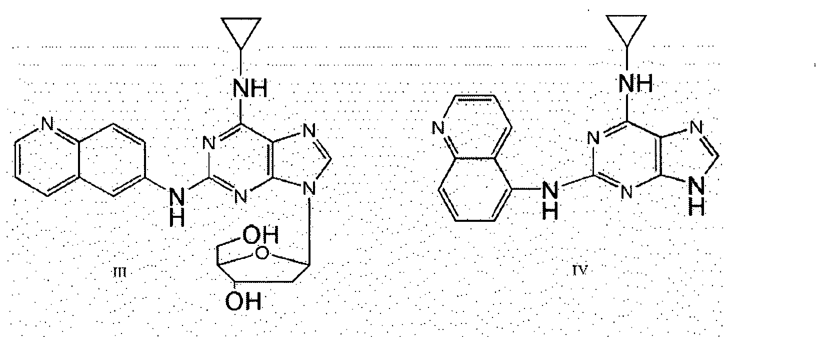

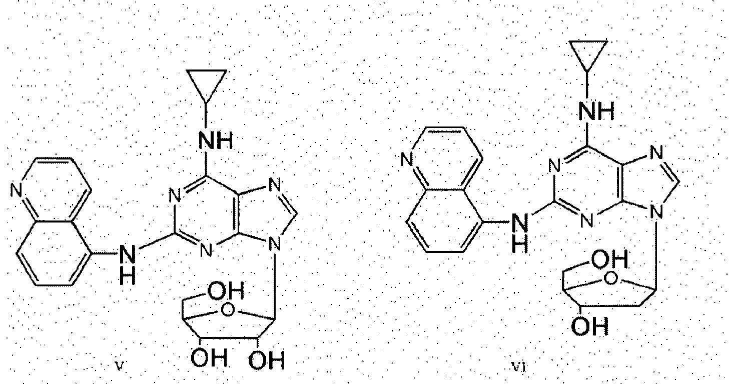

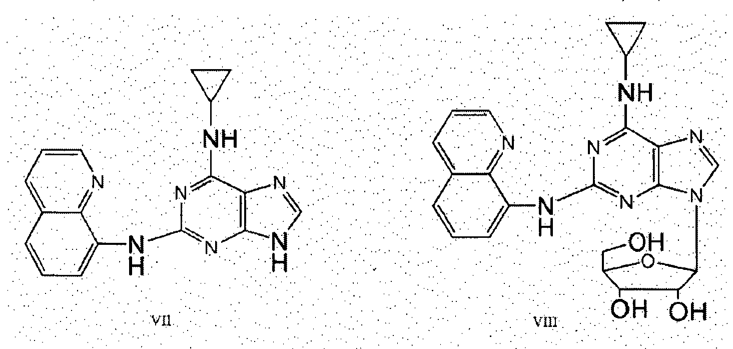

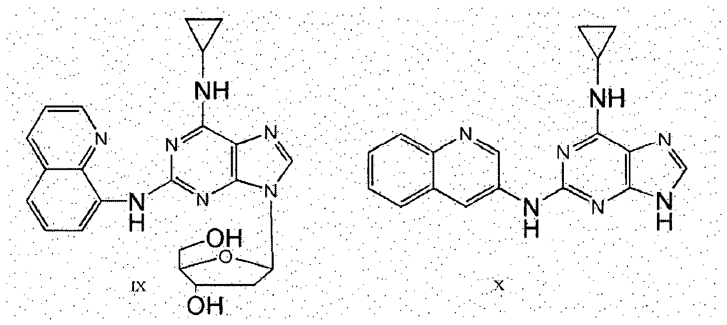

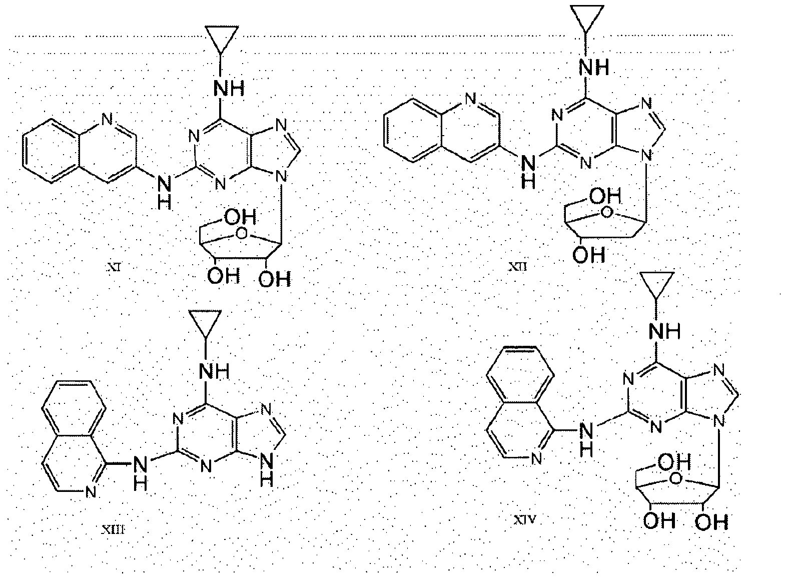

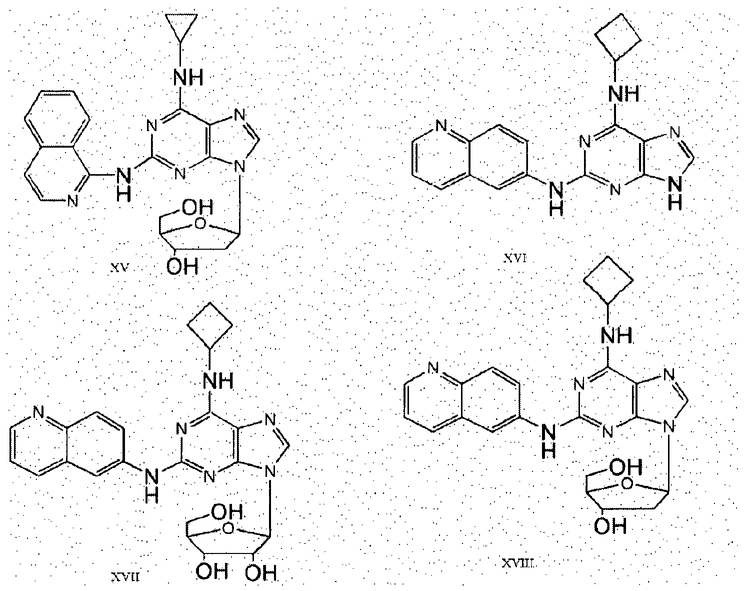

7. Verbindung der Formel (A), ihre Salze, oder Hydrate nach Anspruch 4, wobei die Verbindung durch nachstehende Formel (I) dargestellt ist:

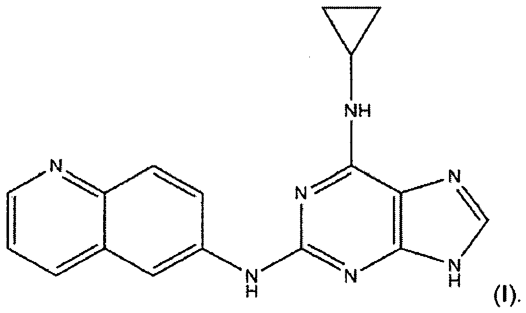

8. Pharmazeutische Zusammensetzung enthaltend eine Verbindung der Formel (A), ihre Salze, oder Hydrate nach einem der Ansprüche 1 bis 7, und einen pharmazeutisch akzeptablen Hilfsstoff.

9. Pharmazeutische Zusammensetzung nach Anspruch 8, wobei die pharmazeutische Zusammensetzung in Form einer Tablette, einer Kapsel, einer Pille, einer oralen flüssigen Zubereitung, eines Granulates, eines Pulvers, einer Injektion, einem Implantat oder als Zubereitung für die externe Verwendung vorliegt.

10. Verfahren zur Herstellung der Verbindung der Formel (A), ihrer Salze, oder Hydrate nach einem der Ansprüche 1 bis 7, wobei das Verfahren die folgenden Schritte umfasst:
(1) Reaktion der Verbindung (j) mit 0,8-1,5 mol/mol Q-NH₂ in einem organischen Lösungsmittel bei einer Temperatur von etwa 50 bis etwa 150°C für 1 - 72 Stunden zu einer ersten Reaktionsmischung, gefolgt von einer Zugabe von Wasser zu der ersten Reaktionsmischung und Abkühlung der ersten Reaktionsmischung bei Raumtemperatur, um eine Verbindung der Formel (b) herzustellen; 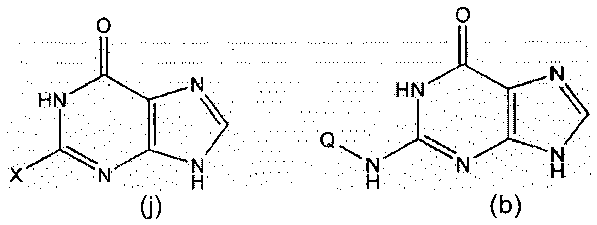
(2) Reaktion der Verbindung nach Formel (b) mit einem halogenisierenden Wirkstoff in einem organischen Lösungsmittel bei einer Temperatur von etwa 50 bis etwa 150°C für 1 - 72 Stunden, Kühlung, gefolgt von einer Zugabe von Wasser zu der Reaktionsmischung und Einstellung des pH-Wertes der Reaktionsmischung auf etwa 2 - 5 mit Säure, und das Abkühlen-lassen der zweiten Reaktionsmischung bei einer Raumtemperatur, um eine Verbindung der Formel (c) herzustellen: 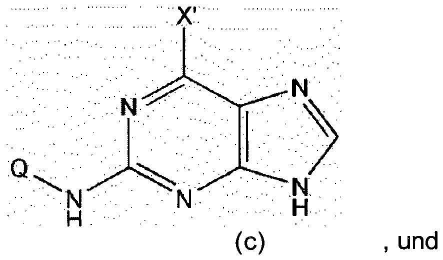
(3) Reaktion der Verbindung (c) mit 0,8-1,5 mol/mol W-NH₂ in einem organischen Lösungsmittel in Anwesenheit eines Säure-Akzeptors bei einer Temperatur von etwa 50 - 150°C für 1 - 72 Stunden, gefolgt von einer Ab-Destillation des Lösungsmittels; um eine Verbindung der Formel (f) zu ergeben; 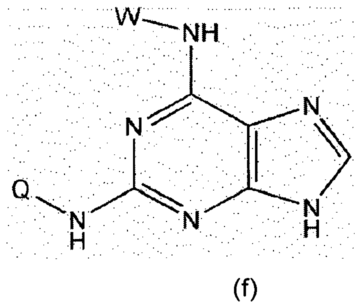 wobei X für Br und X' für Cl steht, und W und Q wie in Anspruch 1 definiert sind.

11. Verfahren zur Herstellung der Verbindung der Formel (A), ihrer Salze, oder Hydrate nach einem der Ansprüche 1 bis 7, wobei das Verfahren die folgenden Schritte umfasst:
(1) Reaktion der Verbindung (k) mit 0,8-1,5 mol/mol W-NH₂ in einem organischen Lösungsmittel in der Anwesenheit eines Säure-Akzeptors bei einer Temperatur von etwa 30 - 120°C für 1 - 72 Stunden gefolgt von einer Ab-Destillation des Lösungsmittels; um eine Verbindung der Formel (e) herzustellen; 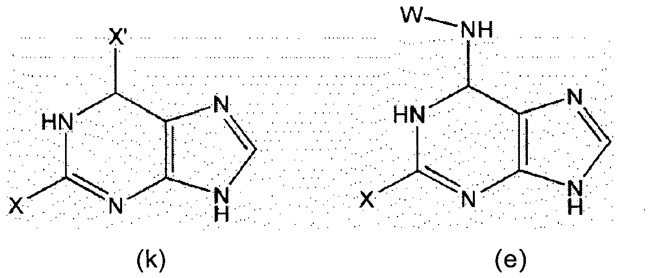
(2) Reaktion der Verbindung nach Formel (e) mit 0,8-1,5 mol/mol Q-NH₂ in einem organischen Lösungsmittel in der Anwesenheit eines Säure-Akzeptors bei einer Temperatur von etwa 70 - 170°C für 1 - 72 Stunden, gefolgt von einer Ab-Destillation des Lösungsmittels; um eine Verbindung der Formel (f) herzustellen; 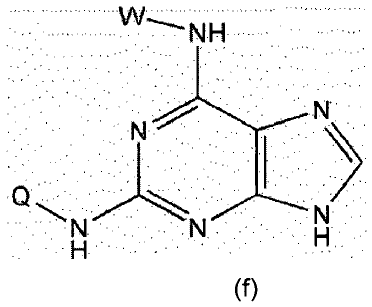 wobei X für Cl und X' für Cl steht, und W und Q wie in Anspruch 1 definiert sind.

12. Verbindung nach Anspruch 1, wobei es sich bei den Salzen um pharmazeutisch akzeptable Salze handelt, bei welchen es sich um zugesetzte Salze handeln kann, hergestellt aus organischen oder anorganischen Säuren, und bei denen es sich vorzugsweise um Hydrochlorid-Salze, Hydrobromid-Salze, Hydrojodat-Salze, p-Toluensulfonat-Salze, Phosphat-Salze, Sulfat-Salze, Perchlorat-Salze, Acetat-Salze, Trifluoracetat-Salze, Propionat-Salze, Citrate, Malonat-Salze, Succinate, Laktat-Salze, Oxalate, Tartrat-Salze und Benzoat-Salze handelt, wobei das Salz auch mit einer Base gebildet werden kann, unter Anderem anorganische und organische Basen.

13. Verwendung einer der Verbindungen, ihrer Salze, oder Hydrate nach den Ansprüchen 1 bis 7 zur Herstellung eines Arzneimittels zur Vorsorge und Behandlung von Tumor-Erkrankungen.

14. Verwendung einer der Verbindungen, ihrer Salze oder Hydrate nach den Ansprüchen 1 bis 7 bei der Herstellung eines Arzneimittels für die Vorsorge und Behandlung von Tumor-Erkrankungen, wobei es sich bei dem Tumor um einen Lungentumor, Lebertumor oder Leukämie handelt.

## Revendications

1. Composé de formule A, ses sels, ou hydrates: 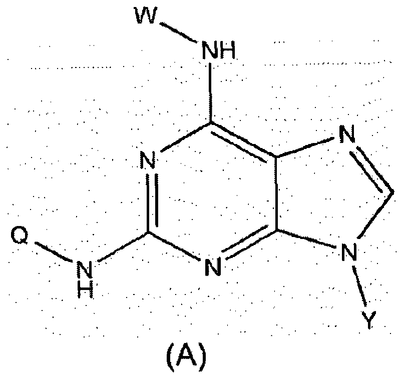 dans lequel
W est un substituent selon une des formules suivantes: 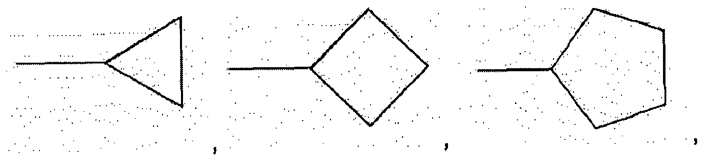 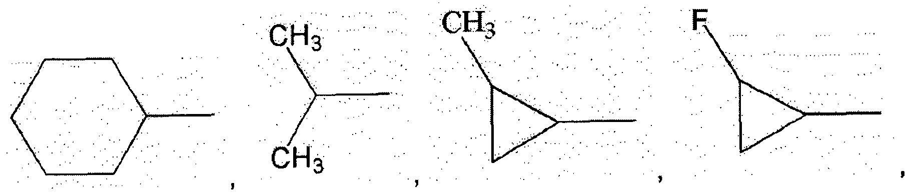 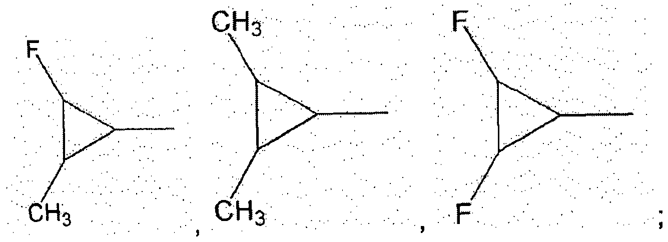
Y est hydrogène, ou un saccharide acceptable en pharmacie selon une des formules suivantes: 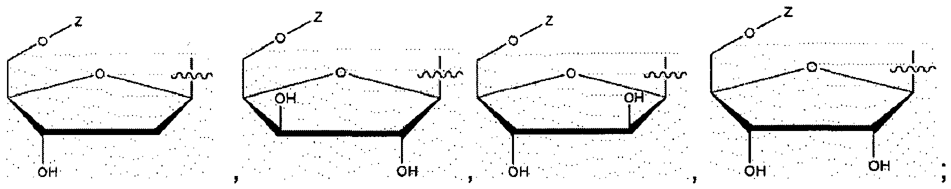
Z est hydrogène, ou un substituent selon une des formules suivantes: 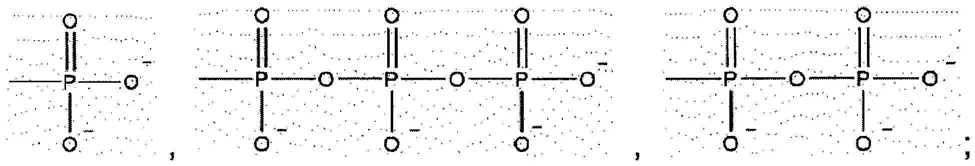
Q est un substituent selon une des formules suivantes: 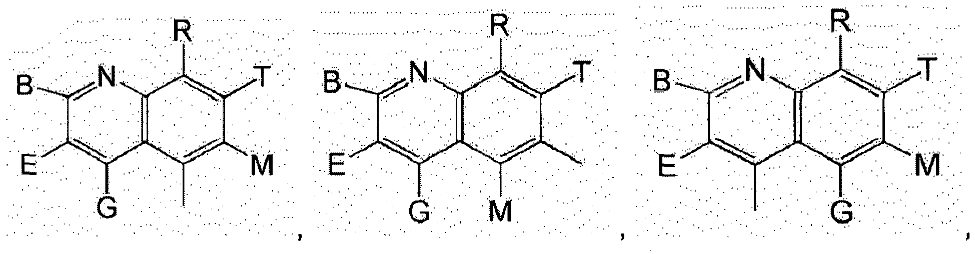 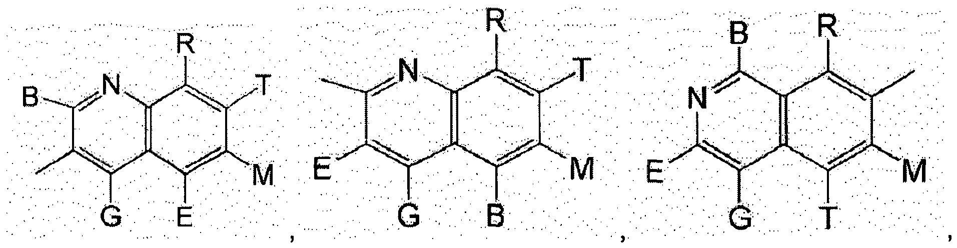 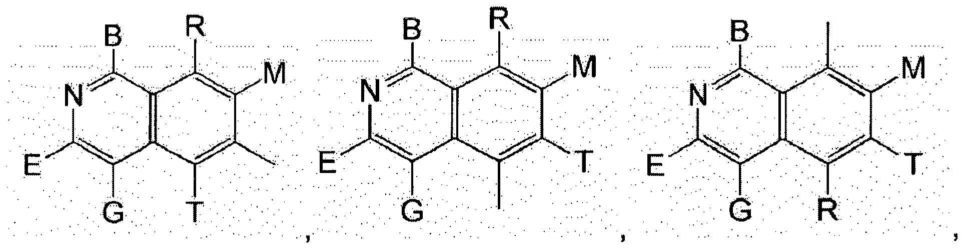 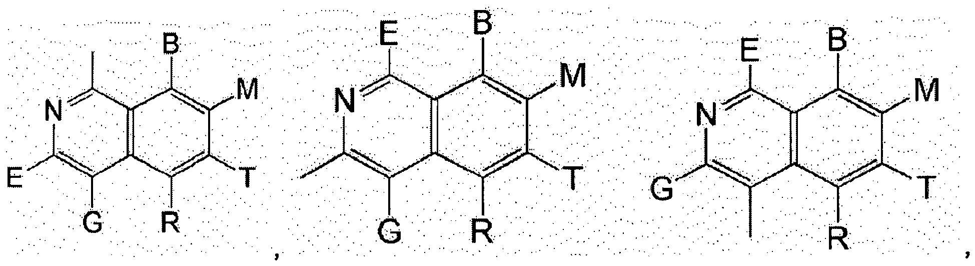 et
B, E, G, R, T, et M sont indépendamment hydrogène, un alkyle (en C₁-C₆) ou un halogénealkyle linéaire ou ramifié, un alkyle cyclique (en C₃-C₆), un groupe halogénique, un groupe cyanogène ou un groupe amino.

2. Composé de formule (A), ses sels, ou hydrates selon la revendication 1, dans lequel Y est un hydrogène.

3. Composé de formule (A), ses sels, ou hydrates selon la revendication 1, dans lequel
W est un substituent selon une des formules suivantes: 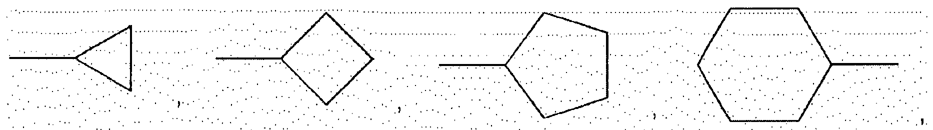 et
B, E, G, R, T, et M sont hydrogène à chaque position.

4. Composé de formule (A), ses sels, ou hydrates selon la revendication 1 ou 2, dans lequel
W est un substituent selon une des formules suivantes: 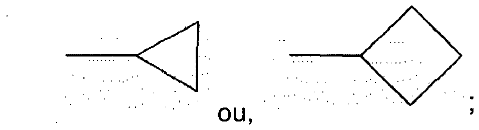
Q est un substituent selon une des formules suivantes:

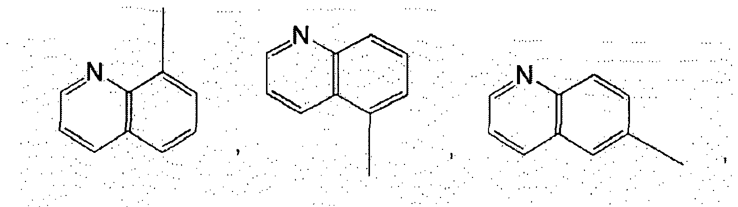

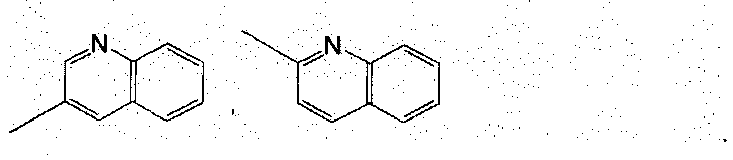

5. Composé de formule (A), ses sels, ou hydrates selon la revendication 4, dans lequel Q est

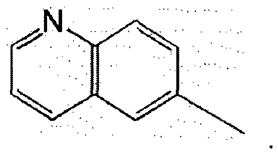

6. Composé de formule (A), ses sels, ou hydrates selon la revendication 1, dans lequel le composé est représenté par une des formules suivantes (I - XVIII):

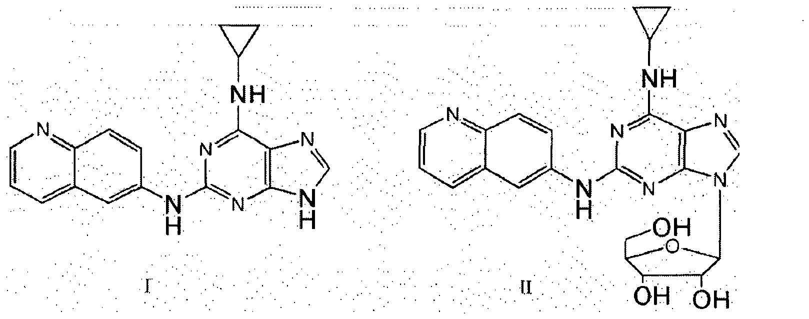

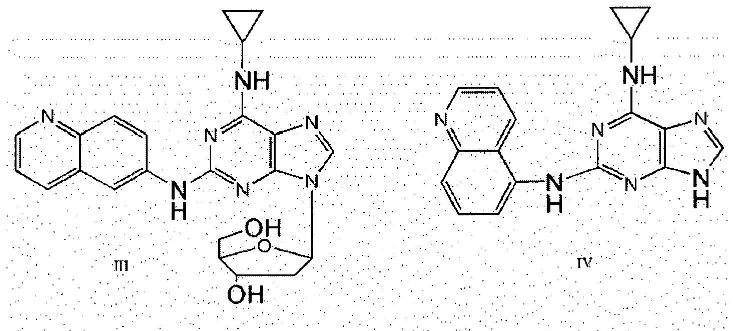

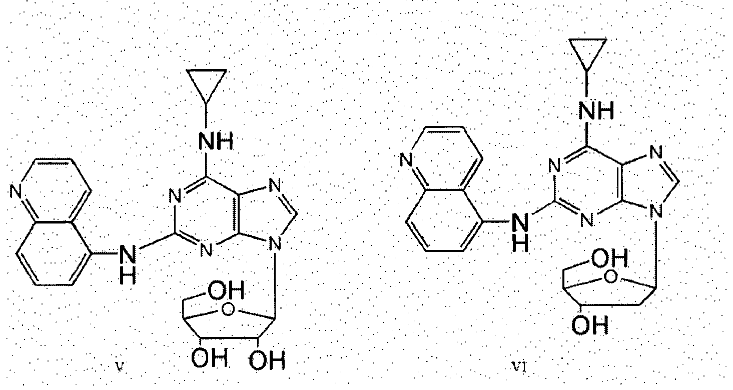

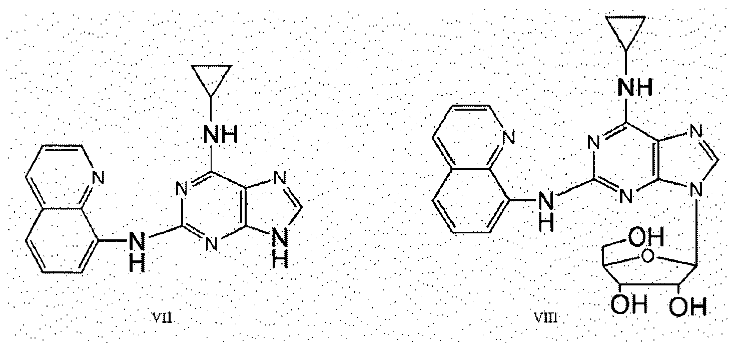

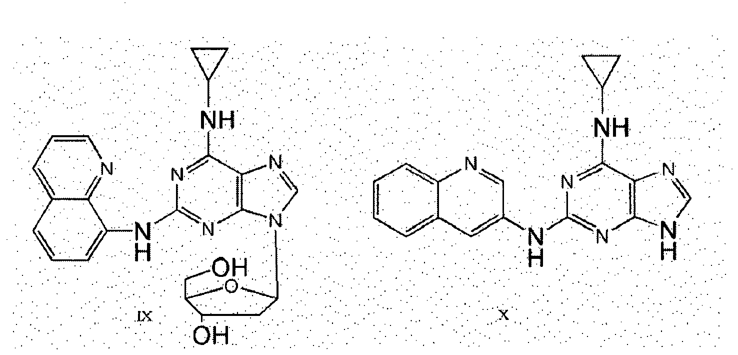

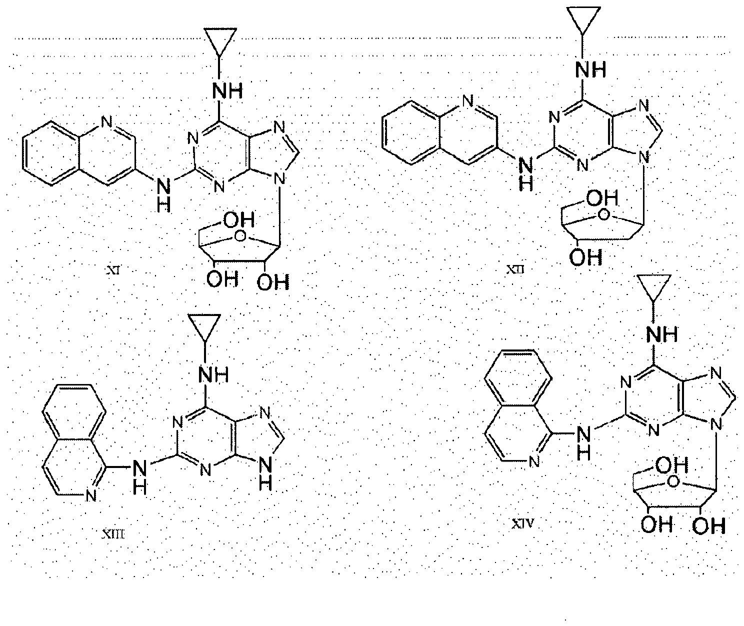

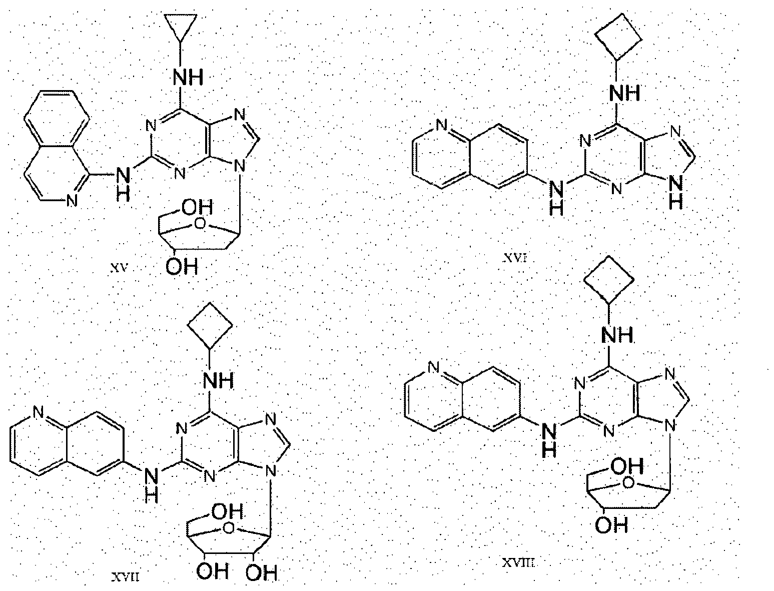

7. Composé de formule (A), ses sels, ou hydrates selon la revendication 4, dans lequel le composé est représenté par la formule suivante (I):

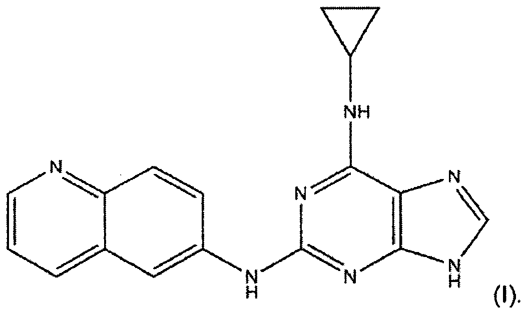

8. Composition pharmaceutique, contenant un composé de formule (A), ses sels, ou hydrates selon l'une quelconque des revendications 1 à 7, et un agent accessoire acceptable en pharmacie.

9. Composition pharmaceutique selon la revendication 8, dans laquelle la composition pharmaceutique est sous forme de comprimés, de capsules, de pilules, de compositions liquides orale, de granulés, d'injections, d'implants ou sous forme de compositions pour utilisation externe.

10. Procédé de préparation d'un composé de formule (A), ses sels, ou hydrates tel que défini dans l'une quelconque des revendications 1 à 7, comprenant:
(1) la mise en réaction d'un composé de formule (j) avec 0,8-1,5 mol/mol Q-NH₂ dans un solvant organique à une température d'environ 50 à environ 150°C pour 1 - 72 heures pour obtenir un premier mélange réactionnel, suivi d'une addition d'eau et refroidissement à température ambiante, pour obtenir un composé de formule (b); 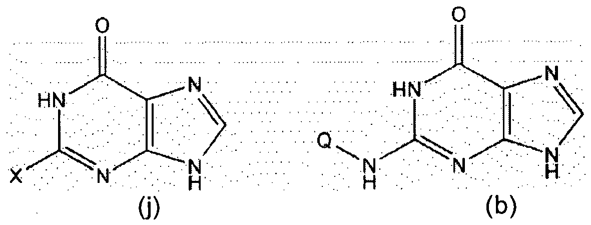
(2) la mise en réaction du composé de formule (b) avec un agent halogénique dans un solvant organique à une température d'environ 50 à environ 150°C pour 1 - 72 heures, refroidissement, suivi d'une addition d'eau et réglage du pH du mélange réactionnel à environ 2 à 5 avec un acide, et le refroidissement du deuxième mélange réactionnel à température ambiante, pour obtenir un composé de formule (c): 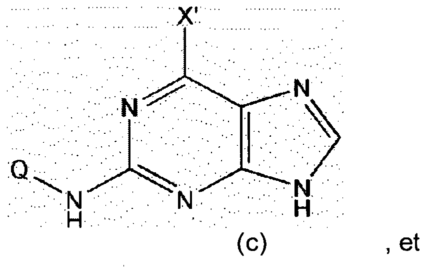
(3) la mise en réaction du composé de formule (c) avec 0,8-1,5 mol/mol W-NH₂ dans un solvant organique en présence d'un accepteur d'acide à une température d'environ 50 à 150°C pour 1 - 72 heures, suivi d'une élimination du solvant par distillation; pour produire un composé de formule (f); 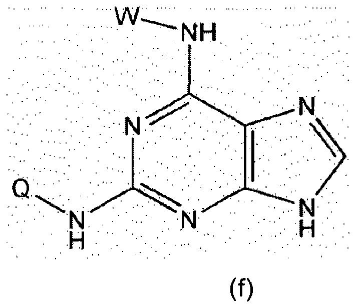 dans lequel X est Br et X' est CI, et W et Q sont tel que défini dans la revendication 1.

11. Procédé de préparation d'un composé de formule (A), ses sels, ou hydrates tel que défini dans l'une quelconque des revendications 1 à 7, comprenant:
(1) la mise en réaction d'un composé de formule (k) avec 0,8-1,5 mol/mol W-NH₂ dans un solvant organique en présence d'un accepteur d'acide à une température d'environ 30 - 120°C pour 1 - 72 heures, suivi d'une élimination du solvant par distillation; pour produire un composé de formule (e); 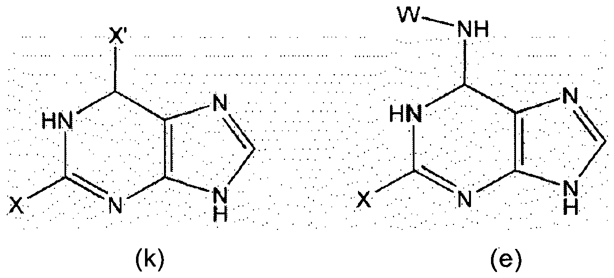
(2) la mise en réaction du composé de formule (e) avec 0,8-1,5 mol/mol Q-NH₂ dans un solvant organique en présence d'un accepteur d'acide à une température d'environ 70 - 170°C pour 1 - 72 heures, suivi d'une élimination du solvant par distillation; pour produire un composé de formule (f); 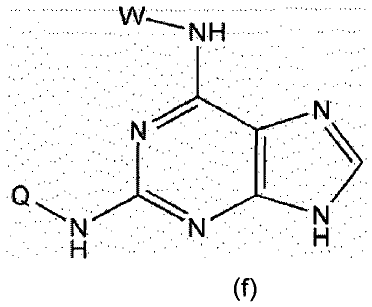 dans lequel X est Cl et X' est Cl, et W et Q sont tel que défini dans la revendication 1.

12. Composé selon la revendication 1, dans lequel les sels sont des sels acceptables en pharmacie, dans lequel les sels peuvent être ajoutés, et sont produits d'acides organique ou anorganique, les sels sont de préférence des sels hydrochlorure, des sels hydrobromure, des sels hydroiodate, des sels de sulfanate p-toluene, des sels de phosphate, des sels de sulfate, des sels de perchlorate, des sels d'acétate, des sels de trifluor acétate, des sels de propionate, des citrates, des sels de malonate, succinate, des sels de lactate, des oxalates, des sels tartrate et benzoate, le sels peut également être produit avec une base, inclus des bases anorganiques et organiques.

13. Utilisation d'un composé, ses sels, ou hydrates selon l'une quelconque des revendications 1 à 7 pour produire un médicament pour la prévention et le traitement des maladies de tumeurs.

14. Utilisation d'un composé, ses sels, ou hydrates selon l'une quelconque des revendications 1 à 7 pour produire un médicament pour la prévention et le traitement des maladies de tumeurs, dans laquelle il s'agit de tumeur de poumon, tumeur de foie ou leucémie.
